# EUROPEAN PATENT APPLICATION

(11) **EP 2 045 246 A1**
(43) Date of publication of application: **08.04.2009**
(21) Application number: 07788618.2
(22) Date of filing: 20.06.2007
(51) Int. Cl.: C07D 275/04, C07D 275/06

(54) **DERIVATIVES OF BENZO[D] ISOTHIAZOLES AS HISTONE DEACETYLASE INHIBITORS**

(30) Priority: 07.07.2006 ES 200601946
(71) Applicant: Universidad De Granada, 18071 Granada (ES)
(72) Inventor: GOMEZ VIDAL, Jose, Antonio, E-18071 Granada (ES); DOMINGUEZ SEGLAR, Jose, Francisco, E-18071 Granada (ES); TABRAUE CHAVEZ, Mavys, E-18071 Granada (ES)
(74) Representative: Carpintero Lopez, Francisco
(86) International application number: PCT/ES2007/000370
(87) International publication number: WO 2008/003800

(57) **Abstract**

The invention relates to derivatives of benzo[d]isothiazoles as histone deacetylase inhibitors, selected from among compounds of general formula (Ia) and (Ib) or one of the salts thereof, particularly one of the pharmaceutically acceptable salts thereof, or one of the corresponding solvates thereof. These compounds are histone deacetylase enzyme inhibitors and are suitable as pharmacologically active agents in a medicament for the treatment and/or prophylaxis of disorders or diseases associated to histone deacetylases. The invention also describes a process for obtaining the mentioned compounds and the pharmaceutical compositions containing them

## Description

### Field of the Art

The present invention relates to new histone deacetylase inhibitor compounds, to new pharmaceutical compositions comprising them, and to processes for obtaining them. These compounds are suitable as pharmacologically active agents in a medicament for the treatment and/or prophylaxis of diseases related to histone deacetylases.

### Background of the Invention

The human genome is located inside the cell nucleus in chromatin, which is a dynamic macromolecular complex formed by nucleosomes. A single nucleosome is made up of a DNA fragment (146 base pairs) coiled around a histone octamer. Histones are small basic proteins rich in the amino acids lysine and arginine. The four types of nucleosomal histones contain two domains: the C-terminal domain, located inside the nucleosome and the N-terminal domain with lysine residues extending outside it.

The acetylation of lysine residues in these N-terminal sequences is mediated by the enzymes called histone acetyltransferases (HATs). The acetyl groups are eliminated from ε-N-acetyl-lysines by the activity of histone deacetylases (HDACs). The activities of HATs and HDACs are associated to the target genes through complexes formed by specific transcription factors for certain sequences and their respective cofactors. The balance between the opposite activities of HATs and HDACs regulates the acetylation state of histones [Marks, P. A.; Richon, V. M.; Rifkind, R. A. Histone deacetylase inhibitors: inducers of differentiation or apoptosis of transformed cells. J. Natl. Cancer Inst. 2000, 92, 1210-1216]. This type of modification regulates key essential processes in the cell in response to extracellular signals [a) Marks, P. A.; Rifkind, R. A.; Richon, V. M.; Breslow, R.; Miller, T.; Kelly, W. K. Histone deacetylases and cancer: causes and therapies. Nature Reviews Cancer 2001, 1(3), 194-202; b) Workman, P. Scoring a bull's-eye against cancer genome targets. Curr. Op. Pharmacol. 2001, 1, 342-352].

High acetylation levels (hyperacetylation) are generally associated to an increase in transcriptional activity, whereas low acetylation levels (hypoacetylation) are associated to the repression of gene expression.

The family of HDACs in mammals includes three sub-classes [Gray, S. G. Ekström, T. J. The human histone deacetylase family. Exp. Cell Res. 2001, 262, 75-83]. Class I includes the HDAC1, HDAC2, HDAC3 and HDAC8 isoforms. Class II includes the HDAC4, HDAC5, HDAC6, HDAC7, HDAC9 and HDAC10 isoenzymes. Finally, class III is homologous to the yeast protein sir2 and includes NAD+-dependent SIRT1-7 isoenzymes and are known as sirtuins. HDAC11 has also been identified as a new member of the family of HDACs, but given the little sequential similarity with the rest, it is not classified within the previous classes. The large number of HDAC isoenzymes and of proteins that interact allows modulating the specificity of the substrate and even modifying the selectivity towards non-histone type targets.

Histone deacetylase enzyme inhibitors which can re-activate gene expression and inhibit tumor cell growth are known in the state of the art, therefore their use in the treatment against cancer is investigated.

Thus, some first-generation histone deacetylase inhibitors are being studied in phase I and II clinical trials [a) Minucci, S.; Pelicci, P. G. Histone deacetylase inhibitors and the promise of epigenetic (and more) treatments for cancer. Nature Reviews Cancer 2006, 6(1), 38-51; b) Johnstone, R. W. Histone-deacetylase inhibitors: novel drugs for the treatment of cancer. Nature Reviews Drug Discovery 2002, 1 (4), 287-299; c) Mai, A.; Massa, S.; Rotili, D.; Cerbara, II.; Valente, S.; Pezzi, R.; Simeoni, S.; Ragno, R. Histone deacetylation in epigenetics: An attractive target for anticancer therapy. Medicinal Research Reviews 2005, 25(3), 261-309]. Due to the fact that the most recently discovered HDAC inhibitors seem to overcome many of the most negative aspects of first-generation inhibitors in clinical use, the therapeutic value derived from the inhibition of HDACs in leukemias and other diseases, including solid and altered hormonal signal-dependent tumors, can be established [Krämer, O. H.; Göttlicher, M.; Heinzel, T. Histone deacetylase as a therapeutic target. Trends Endocrinol. Metabol. 2001, 12, 294-300].

Although this type of inhibitors was initially developed for the treatment of cancer, its use has been proposed in another king of proliferative-type diseases, such as psoriasis [McLaughlin, F.; La Thangue, N. B. Histone deacetylase inhibitors in psoriasis therapy. Current Drug Targets: Inflammation & Allergy 2004, 3(2), 213-219].

The use of this type of inhibitors in the treatment of inflammatory type diseases has also been described [Blanchard, F.; Chipoy, C. Histone deacetylase inhibitors: new drugs for the treatment of inflammatory diseases? Drug Discovery Today 2005, 10(3), 197-204].

A combined therapy with histone deacetylase inhibitors in the treatment against HIV has recently been proposed [Imai, K.; Okamoto, T. Transcriptional Repression of Human Immunodeficiency Virus Type 1 by AP-4. Journal of Biological Chemistry 2006, 281(18), 12495-12505].

Histone deacetylase inhibitors are also useful for the treatment of Alzheimer's disease and dementia [Beglopoulos, V.; Shen, J. Regulation of CRE-dependent transcription by presenilins: prospects for therapy of Alzheimer's disease. Trends in Pharmacological Sciences 2006, 27(1), 33-40].

It would therefore be desirable to identify new histone deacetylase enzyme inhibitor compounds for their use in the treatment or prophylaxis of diseases in which the inhibition of said HDAC enzymes is involved.

The present invention faces the problem of providing alternative histone deacetylase inhibitors to those existing in the state of the art. It has surprisingly been discovered that hydroxamic acid derivative compounds of general formula (Ia) or (Ib) set forth below have a good affinity for histone deacetylases, causing their inhibition. Therefore, these compounds are particularly suitable as pharmacologically active agents in a medicament for the treatment and/or prophylaxis of disorders or diseases sensitive to the inhibition of histone deacetylase enzymes.

### Object of the Invention

In one aspect, the present invention provides histone deacetylase inhibitor compounds selected from among compounds of general formula (Ia) and (Ib) 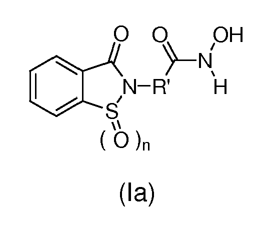 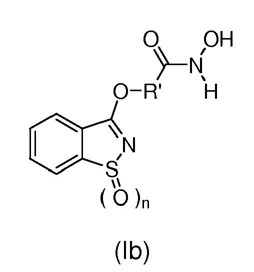 wherein
R' represents a -(CH₂)ₘ- radical, wherein m is 4, 5 or 6, or a 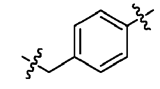 radical and
n is 0 or 2.
optionally in the form of one of the salts thereof, particularly one of the pharmaceutically acceptable salts thereof, or one of the corresponding solvates thereof.

The histone deacetylase inhibitor compounds of general formula (Ia) or (Ib) have affinity for histone deacetylase enzymes and are inhibitors thereof. They are useful in the production of medicaments which are suitable for the treatment and/or prophylaxis of disorders or diseases sensitive to the inhibition of histone deacetylases.

Therefore, in another additional aspect, the invention provides a pharmaceutical composition comprising at least one histone deacetylase inhibitor compound of general formula (Ia) or (Ib) or one of the pharmaceutically acceptable salts thereof, or one of the corresponding solvates thereof.

Likewise, the present invention provides a histone deacetylase inhibitor compound of general formula (Ia) or (Ib) or one of the pharmaceutically acceptable salts thereof, or one of the corresponding solvates thereof for the treatment and/or prophylaxis of diseases sensitive to the inhibition of histone deacetylases in mammals, including man.

In an additional aspect, the present invention provides a histone deacetylase inhibitor compound of general formula (Ia) or (Ib) or one of the pharmaceutically acceptable salts thereof, or one of the corresponding solvates thereof, for the treatment and/or prophylaxis of cancer, inflammatory type diseases, psoriasis, Alzheimer's disease, senile dementia or infection caused by the human immunodeficiency virus (HIV) in mammals, including man.

In another aspect, the present invention provides the use of a histone deacetylase inhibitor compound of general formula (Ia) or (Ib) or one of the pharmaceutically acceptable salts thereof, or one of the corresponding solvates thereof, in the production of a medicament for the treatment and/or prophylaxis of diseases sensitive to the inhibition of histone deacetylases. In an additional aspect, the present invention provides the use of a histone deacetylase inhibitor compound of general formula (Ia) or (Ib) or one of the pharmaceutically acceptable salts thereof, or one of the corresponding solvates thereof, in the production of a medicament for the treatment and/or prophylaxis of cancer, inflammatory type diseases, psoriasis, Alzheimer's disease, senile dementia or the infection caused by the human immunodeficiency virus (HIV) in mammals, including man.

In a final aspect, the present invention provides processes for preparing a histone deacetylase inhibitor compound of general formula (Ia) or (Ib) as has been described above.

### Description of the Invention

In a first aspect, the present invention provides a histone deacetylase inhibitor compound selected from among the compounds of general formula (Ia) and (Ib) 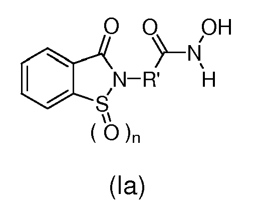 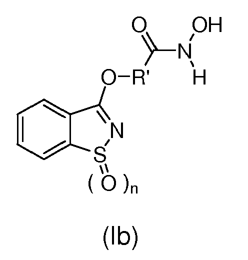 wherein
R' represents a -(CH₂)ₘ- radical, wherein m is 4, 5 or 6, or a 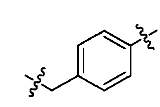 radical, and
n is 0 or 2,
optionally in the form of one of the salts thereof, particularly one of the pharmaceutically acceptable salts thereof, or one of the corresponding solvates thereof.

Therefore, comprised within this first aspect of the invention there are included the salts of the histone deacetylase inhibitor compounds of general formula (Ia) or (Ib), particularly the pharmaceutically acceptable salts and the solvates of the histone deacetylase inhibitor compounds of general formula (Ia) or (Ib) and of the salts thereof, particularly of the pharmaceutically acceptable salts thereof.

The histone deacetylase inhibitor compounds of general formula (Ia) or (Ib), hereinafter compounds of the invention, have affinity for histone deacetylase enzymes and are inhibitors thereof. They are therefore useful in the production of medicaments suitable for the treatment and/or prophylaxis of disorders or diseases sensitive to the inhibition of histone deacetylases. In the context of the present invention, disorders or diseases sensitive to the inhibition of histone deacetylases relate to the disorders or diseases in which the inhibition of histone deacetylases prevents the onset of said disorder or disease or achieves that a mammal's, including a man's, health recovers or improves, from a pathological condition. Proliferative type diseases such as cancer, particularly leukemia, solid and altered hormonal signal-dependent tumors and psoriasis, inflammatory type diseases, the infection caused by HIV, Alzheimer's disease and senile dementia, among others, can be mentioned among said disorders or diseases.

In a particular embodiment of the compounds of the invention, in the compounds of formula (Ia), R' represents a -(CH₂)ₘ- radical, wherein m is 5 or 6, or a 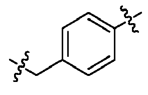 radical, and n is 0 or 2.

In another preferred embodiment of the compounds of the invention, in the compounds of formula (Ia), R' represents a -(CH₂)ₘ- radical, wherein m is 5 or 6 and n is 0 or 2. In an even more preferred embodiment, in the compounds of formula (Ia), R' represents a -(CH₂)ₘ- radical, wherein m is 5 or 6 and n is 2.

In another preferred embodiment of the compounds of the invention, in the compounds of formula (Ia), R' represents a 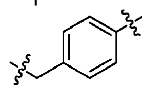 radical, and n is 0 or 2.

In another particular embodiment of the compounds of the invention, in the compounds of formula (Ib), R' represents a -(CH₂)ₘ- radical, wherein m is 4 or 5, or a 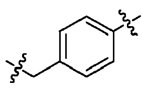 radical, and n is 0 or 2.

In another preferred embodiment of the compounds of the invention, in the compounds of formula (Ib), R' represents a -(CH₂)ₘ- radical, wherein m is 4 or 5 and n is 0 or 2. In an even more preferred embodiment, in the compounds of formula (Ib), R' represents a -(CH₂)ₘ- radical, wherein m is 4 or 5 and n is 0.

In another preferred embodiment of the compounds of the invention, in the compounds of formula (Ib), R' represents a 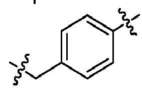 radical, and n is 0 or 2.

In another particular embodiment, the compounds of the invention are selected from the following group:
[1] 6-(1,1-dioxide-3-oxo-2H-benzo[*d*]isothiazol-2-yl)-N-hydroxyhexanamide (MTC-97)
[2] 7-(1,1-dioxide-3-oxo-2H-benzo[*d*]isothiazol-2-yl)-N-hydroxyheptanamide (MTC-124)
[3] 4-[(1,1-dioxide-3-oxo-2H-benzo[*d*]isothiazol-2-yl)methyl]-*N-*hydroxybenzamide (MTC-128)
[4] 6-(3-oxo-2H-benzo[*d*]isothiazol-2-yl)-*N-*hydroxyhexanamide (MTC-136)
[5] 6-[(benzo[*d*]isothiazol-3-yl)oxa]-*N-*hydroxyhexanamide (MTC-144)

In another additional aspect, the invention provides a pharmaceutical composition comprising one or more histone deacetylase inhibitor compounds of general formula (Ia) or (Ib), or one of the pharmaceutically acceptable salts thereof, or one of the corresponding solvates thereof. The pharmaceutical composition of the present invention additionally comprises one or more pharmaceutically acceptable excipients for its administration, such as filler agents, solvents, diluents, coloring agents, coating agents, binders. The choice of the conventional excipients as well as the amount thereof depends on the route of administration intended and can easily be determined by the person skilled in the art. The pharmaceutical composition can be administered, among other routes, by rectal, parenteral, oral, buccal, topical, or inhalatory route. The pharmaceutical compositions provided by the present invention include, for example, tablets, sugar-coated tablets, capsules or multiparticulates such as pellets or granules, solutions, suspensions or suitable liquids, reconstitutable dry preparations, and also preparations for spraying. Likewise, said compositions can be delayed-release compositions generally known in the state of the art or can comprise an enteric coating.

Likewise, the present invention provides a histone deacetylase inhibitor compound of general formula (Ia) or (Ib), or one of the pharmaceutically acceptable salts thereof, or one of the corresponding solvates thereof, for the treatment and/or prophylaxis of diseases sensitive to the inhibition of histone deacetylases in mammals, including man.

In an additional aspect, the present invention provides a histone deacetylase inhibitor compound of general formula (Ia) or (Ib), or one of the pharmaceutically acceptable salts thereof, or one of the corresponding solvates thereof, for the treatment and/or prophylaxis of cancer, particularly leukemia, solid and altered hormonal signal-dependent tumors and psoriasis, inflammatory type diseases, the infection caused by HIV, Alzheimer's disease and senile dementia in mammals, including man.

In another aspect, the present invention provides the use of a histone deacetylase inhibitor compound of general formula (Ia) or (Ib), or one of the pharmaceutically acceptable salts thereof, or one of the corresponding solvates thereof, in the production of a medicament for the treatment and/or prophylaxis of diseases sensitive to the inhibition of histone deacetylases. In an additional aspect, the present invention provides the use of a histone deacetylase inhibitor compound of general formula (Ia) or (Ib), or one of the pharmaceutically acceptable salts thereof, or one of the corresponding solvates thereof, in the production of a medicament for the treatment and/or prophylaxis of cancer, particularly leukemia, solid and altered hormonal signal-dependent tumors and psoriasis, inflammatory type diseases, the infection caused by HIV, Alzheimer's disease and senile dementia in mammals, including man.

In a final aspect the present invention provides a process, which is shown in the following Scheme 1, for preparing a compound of general formula (Ia), 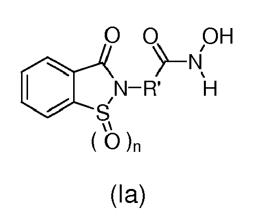 wherein
R' is a -(CH₂)ₘ- radical, n is 0 and m is 5 or 6

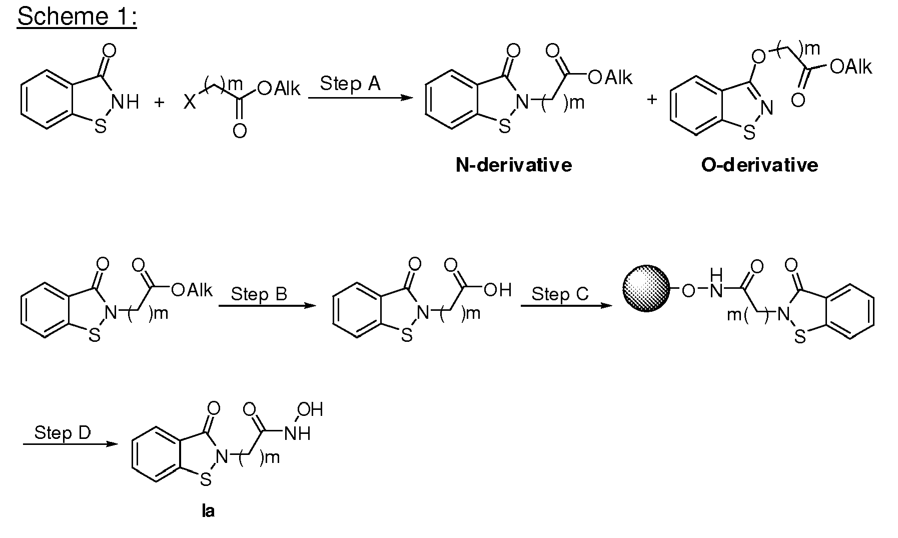

Said process comprises the following reaction steps A, B, C and D described below.

### Step A:

it comprises reacting a derivative of benzo[d]isothiazole, 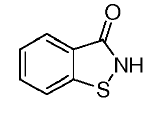 with a compound of formula 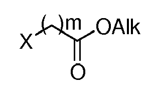 wherein
m is 5 or 6;
X represents a leaving group, such as for example, Br, Cl, -OSO₂CH₃ or a -OSO₂Ph(pCH₃); and
Alk represents an alkyl group such as for example an ethyl, methyl or t-butyl, or an acid function protecting group;
in the presence of an inorganic base and in an inert solvent.

### Step B:

it comprises the hydrolysis of the ester derivative obtained in step A of formula: 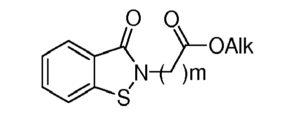 or alternatively, the removal of the acid function protecting group to obtain the corresponding carboxylic acid derivative of formula:

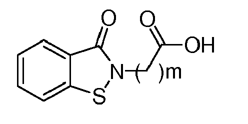

### Step C:

it comprises contacting a Wang resin functionalized with hydroxylamine, with hydroxyazabenzotriazole (HOAt), diisopropylcarbodiimide (DPICDI) and the carboxylic acid derivative obtained in step B to obtain the corresponding hydroxamic acid derivative bound to the Wang resin of formula: 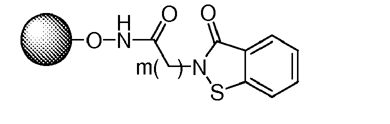 and

### Step D:

it comprises the release of the hydroxamic acid derivative bound to the Wang resin obtained in step C to obtain a compound of general formula (Ia) 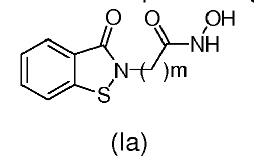 wherein m has the previously defined meaning.

The invention also relates to another process, which is shown in the following Scheme 2, for preparing a compound of general formula (Ia), 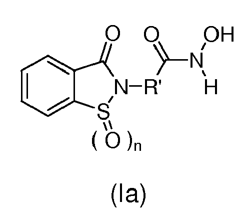 wherein
R' is a -(CH₂)ₘ- radical, n is 2 and m is 5 or 6

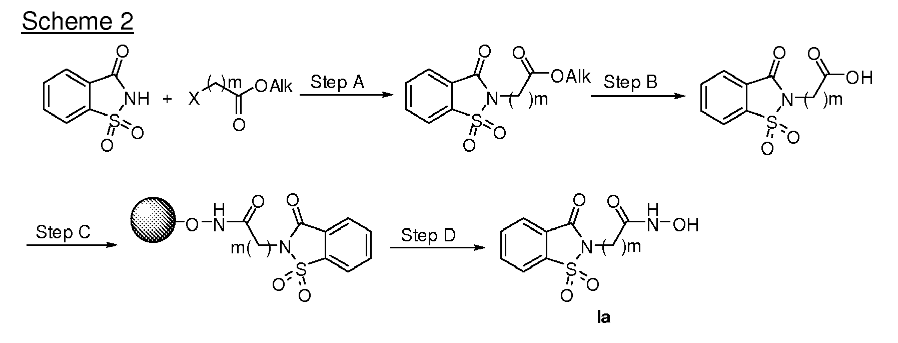

Said process comprises the following reaction steps A, B, C and D which are described below.

### Step A:

it comprises reacting a derivative of benzo[d]isothiazole, 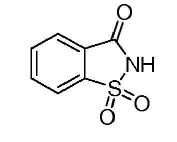 with a compound of formula 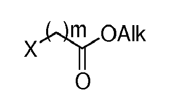 wherein
m is 5 or 6;
X represents a leaving group, such as for example, Br, Cl, -OSO₂CH₃ or a -OSO₂Ph(pCH₃); and
Alk represents an alkyl group such as for example an ethyl, methyl or t-butyl, or an acid function protecting group;
in the presence of an inorganic base and in an inert solvent.

### Step B:

it comprises the hydrolysis of the ester derivative obtained in step A of formula: 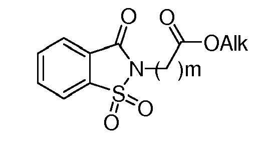 or alternatively, the removal of the acid function protecting group to obtain the corresponding carboxylic acid derivative of formula:

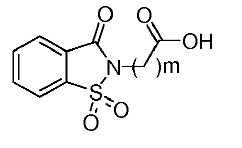

### Step C:

it comprises contacting a Wang resin functionalized with hydroxylamine, with hydroxyazabenzotriazole (HOAt), diisopropylcarbodiimide (DPICDI) and the carboxylic acid derivative obtained in step B to obtain the corresponding hydroxamic acid derivative bound to the Wang resin of formula: 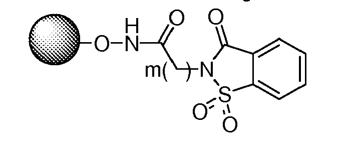 and

### Step D:

it comprises the release of the hydroxamic acid derivative bound to the Wang resin obtained in step C to obtain a compound of general formula (Ia) 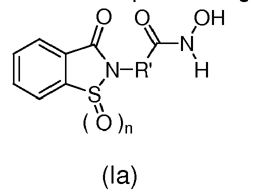 wherein R' and n have the previously defined meaning.

The invention also relates to another process, which is shown in the following Scheme 3, for preparing a compound of general formula (Ia), 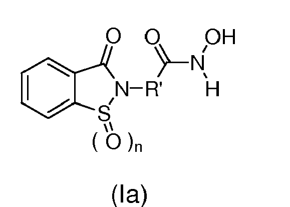 wherein R' is a 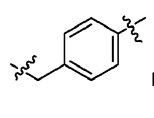 radical, and n is 0.

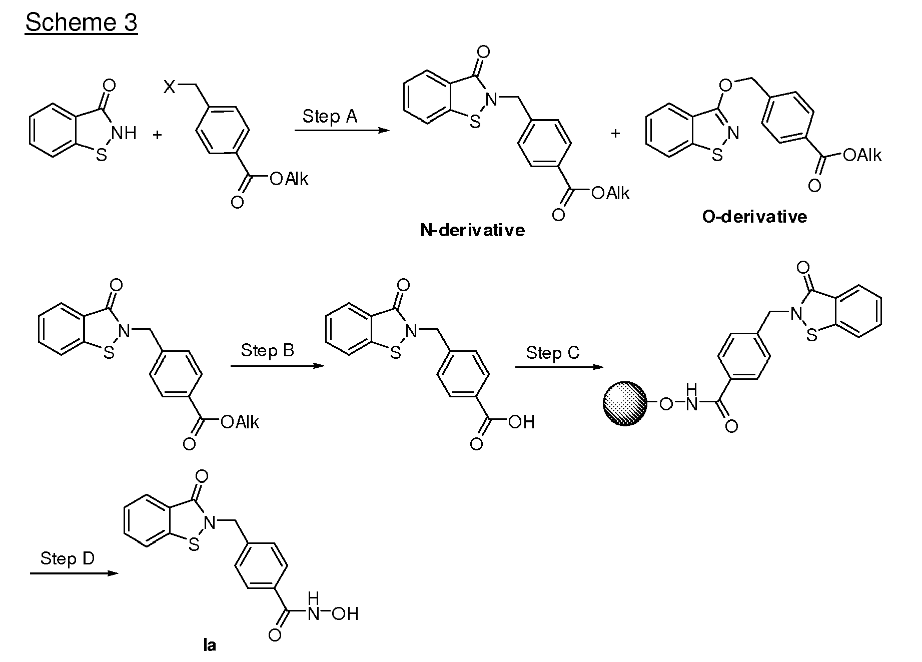

Said process comprises the following reaction steps A, B, C and D which are described below.

### Step A:

it comprises reacting a derivative of benzo[d]isothiazole, 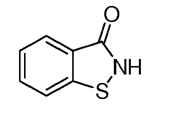 with a compound of formula: 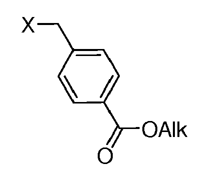 wherein
X represents a leaving group, such as for example, Br, Cl, -OSO₂CH₃ or a - OSO₂Ph(pCH₃); and
Alk represents an alkyl group such as for example, ethyl, methyl or t-butyl, or an acid function protecting group;
in the presence of an inorganic base and in an inert solvent.

### Step B:

it comprises the hydrolysis of the ester derivative obtained in step A of formula: 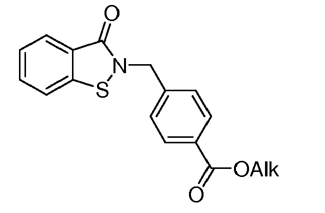 or alternatively, the removal of the acid function protecting group to obtain the corresponding carboxylic acid derivative of formula:

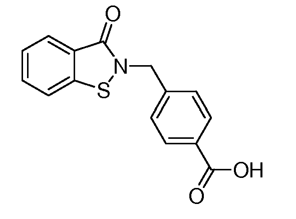

### Step C:

it comprises contacting a Wang resin functionalized with hydroxylamine, with hydroxyazabenzotriazole (HOAt), diisopropylcarbodiimide (DPICDI) and the carboxylic acid derivative obtained in step B to obtain the corresponding hydroxamic acid derivative bound to the Wang resin of formula: 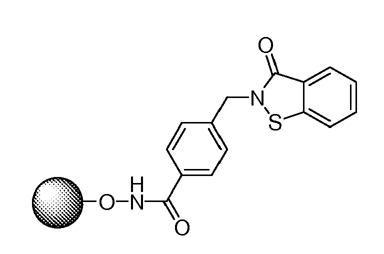 ; and

### Step D:

it comprises the release of the hydroxamic acid derivative bound to the Wang resin obtained in step C to obtain a compound of general formula (Ia) 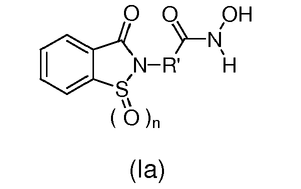 wherein R' and n have the previously defined meaning.

In the previously defined processes for preparing compounds of general formula (Ia) of the invention, the reaction conditions of Steps A, B, C and D are common.

The invention also relates to another process, which is shown in the following Scheme 4, for preparing a compound of general formula (Ia), 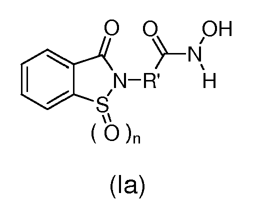 wherein R' is a 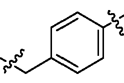 radical, and n is 2.

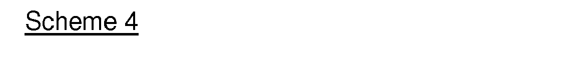

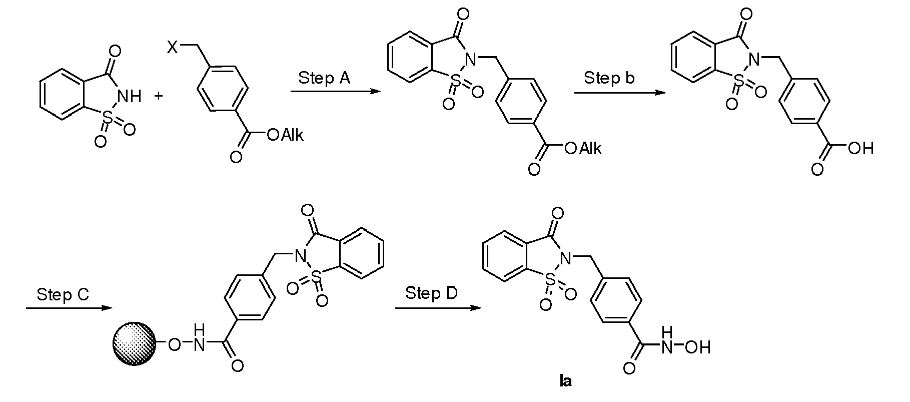

Said process comprises the following reaction steps A, B, C and D which are described below.

### Step A:

it comprises reacting a derivative of benzo[d]isothiazole, 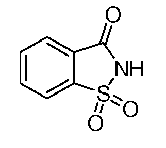 with a compound of formula: 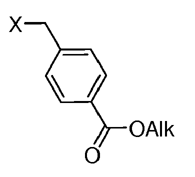 wherein
X represents a leaving group, such as for example, Br, Cl, -OSO₂CH₃ or a - OSO₂Ph(pCH₃); and
Alk represents an alkyl group such as for example, ethyl, methyl or t-butyl, or an acid function protecting group;
in the presence of an inorganic base and in an inert solvent.

### Step B:

it comprises the hydrolysis of the ester derivative obtained in step A of formula: 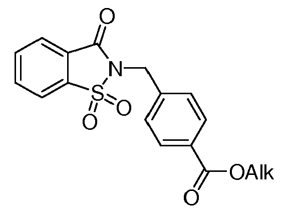 or alternatively, the removal of the acid function protecting group to obtain the corresponding carboxylic acid derivative of formula:

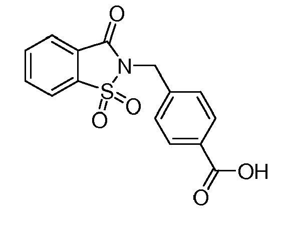

### Step C:

it comprises contacting a Wang resin functionalized with hydroxylamine, with hydroxyazabenzotriazole (HOAt), diisopropylcarbodiimide (DPICDI) and the carboxylic acid derivative obtained in step B to obtain the corresponding hydroxamic acid derivative bound to the Wang resin of formula: 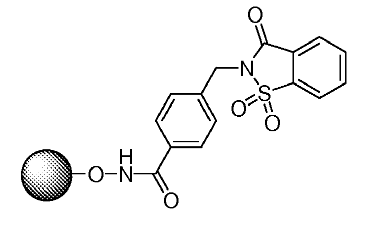 ; and

### Step D:

it comprises the release of the hydroxamic acid derivative bound to the Wang resin obtained in step C to obtain a compound of general formula (Ia) 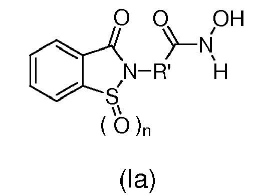 wherein R' and n have the previously defined meaning.

In the previously defined processes for preparing compounds of general formula (Ia) of the invention, the reaction conditions of Steps A, B, C and D are common.

The invention also relates to another process, which is shown in the following Scheme 5, for preparing a compound of general formula (Ib), 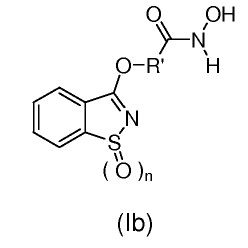 wherein
R' is a -(CH₂)ₘ- radical, m is 4 or 5, and n is 0.

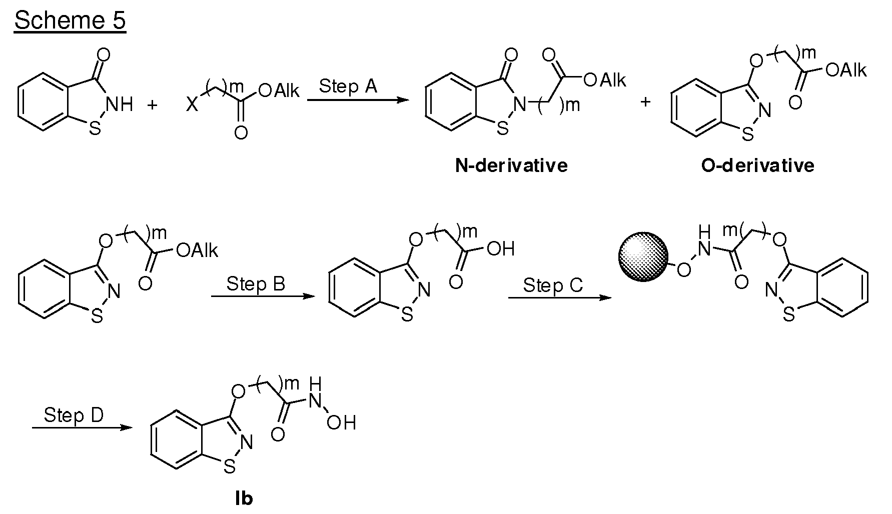

Said process comprises the following reaction steps A, B, C and D which are described below.

### Step A:

it comprises reacting a derivative of benzo[d]isothiazole, 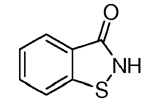 with a compound of formula 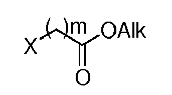 wherein
m is 4 or 5;
X represents a leaving group, such as for example, Br, Cl, -OSO₂CH₃ or a - OSO₂Ph(pCH₃); and
Alk represents an alkyl group such as for example an ethyl, methyl or t-butyl, or an acid function protecting group;
in the presence of an inorganic base and in an inert solvent.

### Step B:

it comprises the hydrolysis of the ester derivative obtained in step A of formula: 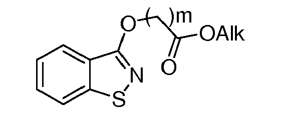 wherein Alk and m have the aforementioned meaning,
or alternatively, the removal of the acid function protecting group to obtain the corresponding carboxylic acid derivative of formula: 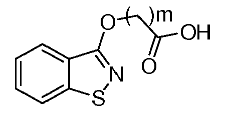 wherein m has the aforementioned meaning

### Step C:

it comprises contacting a Wang resin functionalized with hydroxylamine, with hydroxyazabenzotriazole (HOAt), diisopropylcarbodiimide (DPICDI) and the carboxylic acid derivative obtained in step B to obtain the corresponding hydroxamic acid derivative bound to the Wang resin of general formula: 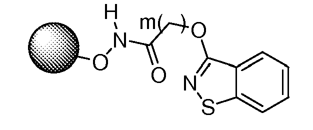 wherein m has the aforementioned meaning; and

### Step D:

it comprises the release of the hydroxamic acid derivative bound to the Wang resin obtained in step C to obtain a compound of general formula (Ib) 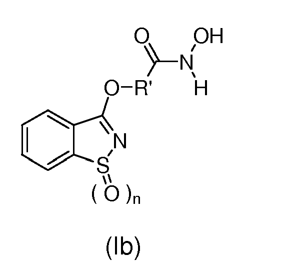 wherein R' and n have the previously defined meaning.

The invention also relates to another process, which is shown in the following Scheme 6, for preparing a compound of general formula (Ib), 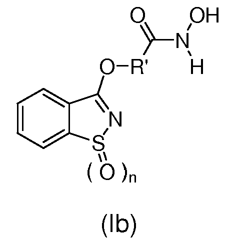 wherein
R' is a -(CH₂)ₘ- radical, m is 4 or 5, and n is 2.

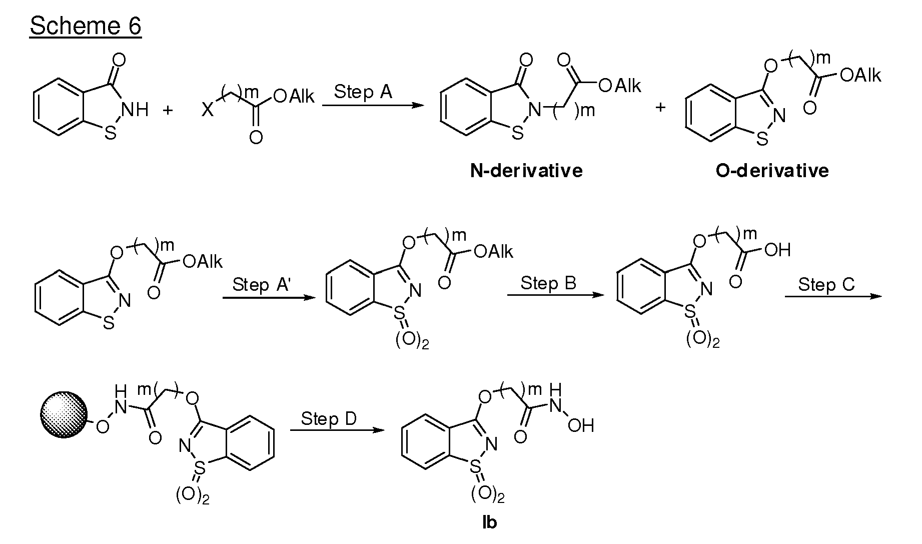

Said process comprises the following reaction steps A, A', B, C and D which are described below.

### Step A:

it comprises reacting a derivative of benzo[d]isothiazole, 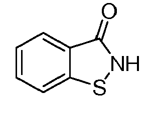 with a compound of formula 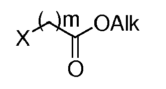 wherein
m is 4 or 5;
X represents a leaving group, such as for example, Br, Cl, -OSO₂CH₃ or a - OSO₂Ph(pCH₃); and
Alk represents an alkyl group such as for example an ethyl, methyl or t-butyl,
or an acid function protecting group;
in the presence of an inorganic base and in an inert solvent.

### Step A':

it comprises the oxidation of the ester obtained in step A, or alternatively the product with the acid function protected, of general formula 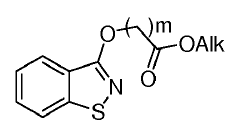 wherein m and Alk have the aforementioned meaning,
in the presence of a solution of H₅lO₆ and CrO₃ in acetonitrile and at -35ºC.

The addition of the solution of H₅lO₆ and CrO₃ in acetonitrile at -35ºC to a solution in ethyl acetate of the previous ester is carried out according to the published process of Xu et al. [Xu, L.; Cheng, J.; Trudell, M. L. Chromonium (VI) oxide catalyzed oxidation of sulfides to sulfones with periodic acid. Journal of Organic Chemistry 2003, 68, 5388-5391].

### Step B:

it comprises the hydrolysis of the oxidized ester derivative obtained in step A' of general formula 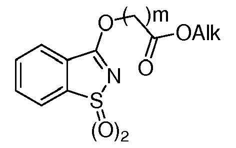 wherein Alk and m have the aforementioned meaning,
or alternatively, the removal of the acid function protecting group to obtain the corresponding carboxylic acid derivative of general formula: 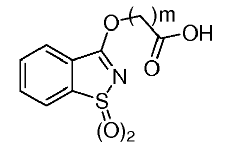 wherein m has the aforementioned meaning

### Step C:

it comprises contacting a Wang resin functionalized with hydroxylamine, with hydroxyazabenzotriazole (HOAt), diisopropylcarbodiimide (DPICDI) and the carboxylic acid derivative obtained in step B to obtain the corresponding hydroxamic acid derivative bound to the Wang resin of general formula: 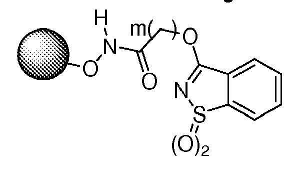 wherein m has the aforementioned meaning; and

### Step D:

it comprises the release of the hydroxamic acid derivative bound to the Wang resin obtained in step C to obtain a compound of general formula (Ib) 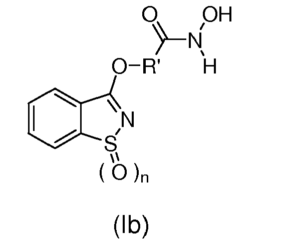 wherein R' and n have the previously defined meaning

The invention also relates to another process, which is shown in the following Scheme 7, for preparing a compound of general formula (Ib), 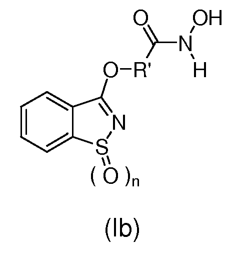 wherein R' is a radical 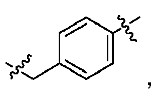 and n is 0.

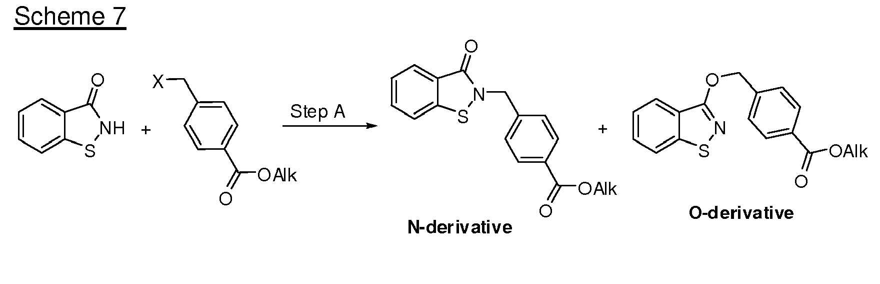

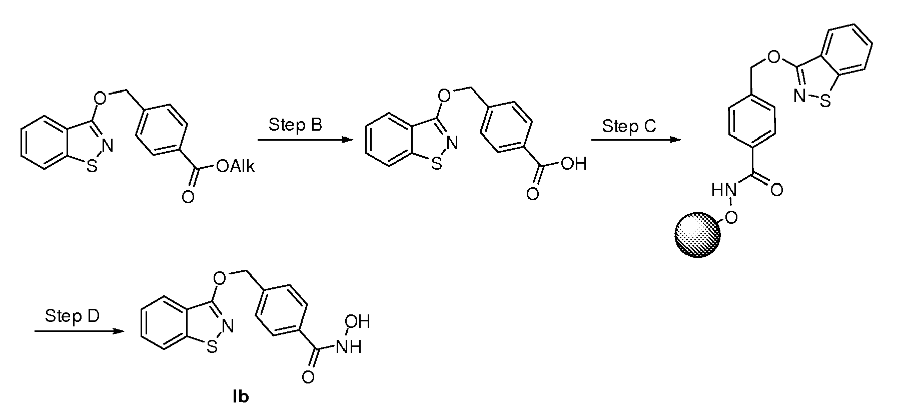

Said process comprises the following reaction steps A, B, C and D which are described below.

### Step A:

it comprises reacting a derivative of benzo[d]isothiazole, 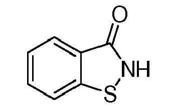 with a compound of formula: 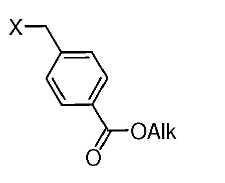 wherein
X represents a leaving group, such as for example, Br, Cl, -OSO₂CH₃ or a - OSO₂Ph(pCH₃); and
Alk represents an alkyl group such as for example, ethyl, methyl or t-butyl, or
an acid function protecting group;
in the presence of an inorganic base and in an inert solvent.

### Step B:

it comprises the hydrolysis of the ester derivative obtained in step A of general formula 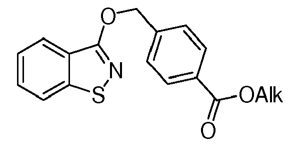 wherein Alk has the aforementioned meaning
or alternatively, the removal of the acid function protecting group to obtain the corresponding carboxylic acid derivative of general formula:

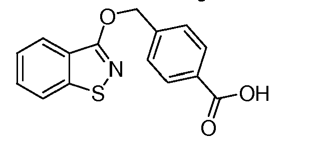

### Step C:

it comprises contacting a Wang resin functionalized with hydroxylamine, with hydroxyazabenzotriazole (HOAt), diisopropylcarbodiimide (DPICDI) and the carboxylic acid derivative obtained in step B to obtain the corresponding hydroxamic acid derivative bound to the Wang resin of general formula: 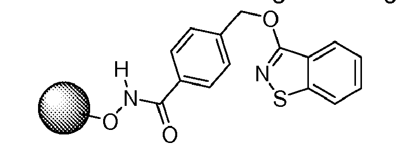 ; and

### Step D:

it comprises the release of the hydroxamic acid derivative bound to the Wang resin obtained in step C to obtain a compound of general formula (Ib) 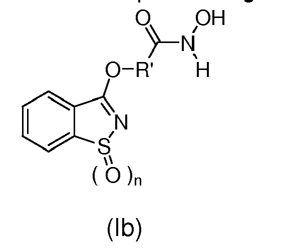 wherein R' and n have the previously defined meaning.

The invention also relates to another process, which is shown in the following Scheme 8, for preparing a compound of general formula (Ib), 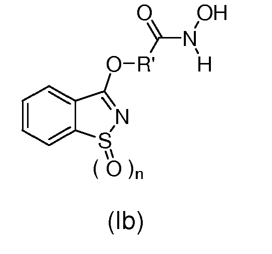 wherein R' is a 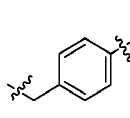 radical, and n is 2.

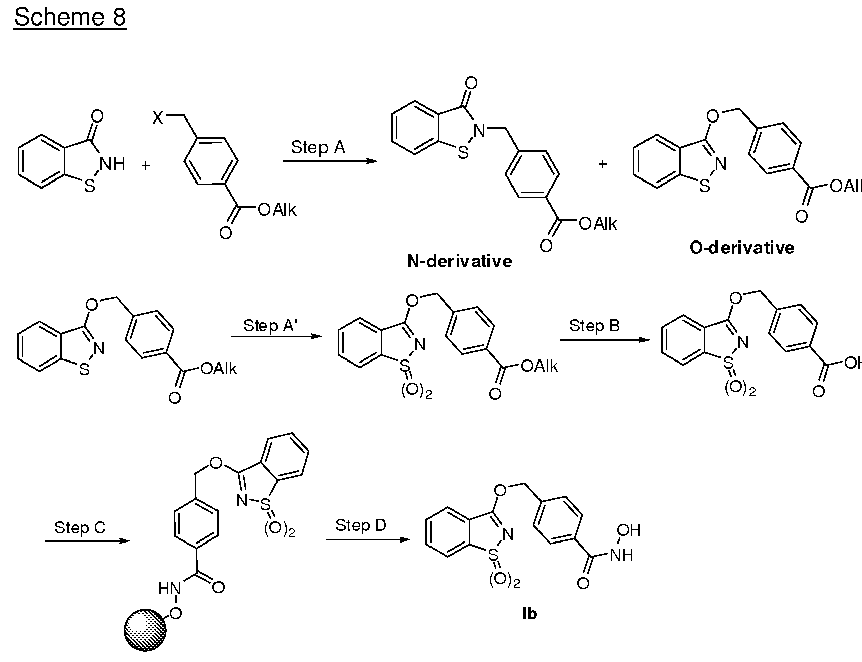

Said process comprises the following reaction steps A, A', B, C and D which are described below.

### Step A:

it comprises reacting a derivative of benzo[d]isothiazole, 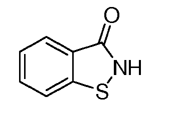 with a compound of formula: 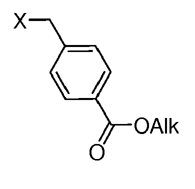 wherein
X represents a leaving group, such as for example, Br, Cl, -OSO₂CH₃ or a - OSO₂Ph(pCH₃); and
Alk represents an alkyl group such as for example, ethyl, methyl or t-butyl, or an acid function protecting group;
in the presence of an inorganic base and in an inert solvent.

### Step A':

it comprises oxidation of the ester obtained in step A, or alternatively the product with the acid function protected, of general formula 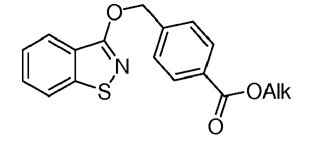 wherein m and Alk have the aforementioned meaning,
in the presence of a solution of H₅lO₆ and CrO₃ in acetonitrile and at -35ºC.

The addition of the solution of H₅lO₆ and CrO₃ in acetonitrile at -35ºC to a solution in ethyl acetate of the previous ester is carried out according to the published process of Xu et al. [Xu, L.; Cheng, J.; Trudell, M. L. Chromonium (VI) oxide catalyzed oxidation of sulfides to sulfones with periodic acid. Journal of Organic Chemistry 2003, 68, 5388-5391].

### Step B

it comprises the hydrolysis of the oxidized ester derivative obtained in step A' of general formula 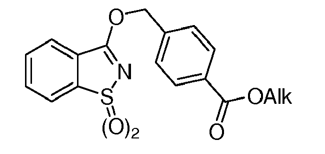 wherein Alk has the aforementioned meaning
or alternatively, the removal of the acid function protecting group to obtain the corresponding carboxylic acid derivative of general formula: 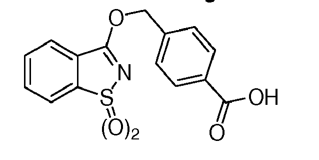 it comprises contacting a Wang resin functionalized with hydroxylamine, with hydroxyazabenzotriazole (HOAt), diisopropylcarbodiimide (DPICDI) and the carboxylic acid derivative obtained in step B to obtain the corresponding hydroxamic acid derivative bound to the Wang resin general formula: 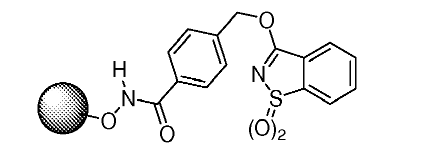 ; and

### Step D:

it comprises the release of the hydroxamic acid derivative bound to the Wang resin obtained in step C to obtain a compound of general formula (Ib) 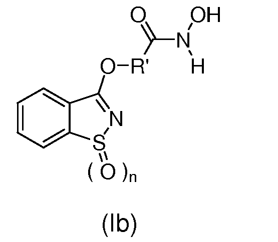 wherein R' and n have the previously defined meaning

In the previously defined processes for preparing compounds of general formula (Ib) of the invention, the reaction conditions of Steps A, A', B, C and D are common.

The starting compounds for step A : 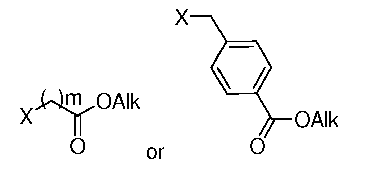 wherein X, m and Alk having the aforementioned meanings are commercially available or can easily be prepared by a person skilled in the art by means of conventional processes.

The starting derivatives of benzo[d]isothiazole, benzo[d]isothiazol-3(2H)-one and 1,1-dioxide-3-oxo-2H-benzo[d]isothiazole, are marketed by the company Sigma-Aldrich.

With respect to the starting compounds of general formulas: 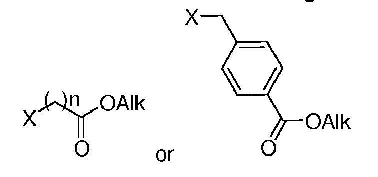 Alk, as previously mentioned, can be an acid function protecting group. Said protecting group can be any conventional protecting group known to a person skilled in the art such as for example an allyl protecting group to form allyl esters. Its deprotection can be carried out by means of conventional methods, for example, in the presence of palladium (0), triphenylphosphine and phenylsilane. Depending on its nature, the conditions for its deprotection in step B of the process are also known to a person skilled in the art.

Step A of the previously defined processes is carried out in the presence of an inorganic base and in an inert solvent. In a particular embodiment, said inorganic base is a carbonate, such as for example potassium carbonate. In a particular embodiment, said inert solvent is dimethylformamide (DMF). The reaction between the starting products is carried out by heating the reaction mixture at a suitable temperature depending on the solvent, typically about 120ºC and for a time which can be variable depending on the starting products, it is typically 24 hours. The intermediate reaction product obtained in step A can be precipitated by adding water-ice, it is filtered, and it is obtained in the form of a solid which can be dried under vacuum. Alternatively, the intermediate reaction products obtained in step A which do not precipitate in the previous conditions can be extracted with a suitable organic solvent, such as for example ethyl acetate. The organic phase is dried, filtered and concentrated in the rotary evaporator. The obtained product can optionally be purified by means of flash chromatography using mixtures of suitable solvents, such as for example ethyl acetate/hexane.

Step A' is carried out when in the compound to be obtained with structure (Ib), n has a value of 2. To that end, on a solution of the O-derivative (obtained in step A) in ethyl acetate, a solution of H₅lO₆ and CrO₃ in acetonitrile, at -35ºC, is added dropwise according to the process described by Xu et al. [Xu, L.; Cheng, J.; Trudell, M. L. Chromonium (VI) oxide catalyzed oxidation of sulfides to sulfones with periodic acid. Journal of Organic Chemistry 2003, 68, 5388-5391].

In step B, the hydrolysis of the product obtained in step A is carried out. In the event that said derivative is an ester derivative, the hydrolysis thereof is typically carried out in the presence of an acid by heating. In a particular embodiment, concentrated hydrochloric acid is used. The intermediate product obtained is precipitated by adding water, it is filtered and optionally purified by means of flash chromatography using mixtures of suitable solvents such as for example dichloromethane/methanol.

In step C, the Wang resin functionalized with hydroxylamine is a commercially available product. It is conditioned with an aprotic polar inert solvent, typically dichloromethane. It is then filtered, and washed with an inert solvent, typically dimethylformamide. HOAt, DIPCDI and the carboxylic acid derivative obtained in step B in a suitable solvent, particularly DMF, is added. Once the reaction between the carboxylic acid derivative and the functionalized resin has ended, the latter is filtered, washed typically with DMF and conditioned with dichloromethane.

In step D, the hydroxamic acid derivative bound to the resin is released by adding an acid in a solvent. In a particular embodiment, TFA in DCM is used. The resulting derivative of general formula (Ia) or (Ib) can be purified and/or isolated according to conventional processes known by persons skilled in the art. In a particular embodiment, it is carried out by means of flash chromatography and is characterized by nuclear magnetic resonance (NMR) and mass spectrometry (MS), particularly high resolution liquid secondary ion mass spectrometry (HR LSIMS).

During one or more steps of the synthesis processes described above and shown in Schemes 1 to 8, it can be necessary and/or desirable to protect sensitive or reactive groups in some of the molecules used. This can be carried out by means of conventional protecting groups such as those described in the literature [a) T. W. Greene & P. G. M. Wuts, Protective Groups in Organic Chemistry, John Wiley & Sons, 3rd edition, 1999; b) Philip J. Kocienski, Protecting Groups, Thieme, 3rd edition, 2004]. The protecting groups can be eliminated in a suitable subsequent step by processes known by persons skilled in the art. The respective literature descriptions are incorporated in the present specification as a reference and form part of the description.

The pharmacologically acceptable salts of the histone deacetylase inhibitor compounds of general formula (Ia) or (Ib) can be prepared by conventional processes known by persons skilled in the art, comprising the reaction with a base to form the corresponding addition salt, for example, ammonium, alkali or alkaline earth salts, particularly lithium, sodium, potassium, magnesium, calcium salts or a salt with an organic base such as benzathine, N-methyl-D-glucamine, or with amino acids such as lysine or arginine.

The pharmaceutically acceptable solvates, particularly hydrates and alcoholates of the derivatives of general formula (Ia) or (Ib) or of the corresponding physiologically acceptable salts thereof can be prepared by conventional processes known by persons skilled in the art.

### Examples

### Example 1. Synthesis of the inhibitor 6-(1,1-dioxide-3-oxo-2H-benzo[d]isothiazol-2-yl)-N-hydroxyhexanamide (MTC-97)

The synthesis comprised the following steps:

### 1.1 Synthesis of ethyl 6-(1,1-dioxide-3-oxo-2H-benzo[d]isothiazol-2-yl)hexanoate (Step A)

The starting materials were 1,1-dioxide-3-oxo-2H-benzo[d]isothiazole (400 mg, 1.95 mmol) and ethyl 6-bromohexanoate (0.35 ml, 1.95 mmol) in 1 ml of DMF. The purification was carried out by means of extracting with ethyl acetate (3x 5 ml), after adding ice-water. The organic phases were dried on MgSO₄, filtered and concentrated in the rotary evaporator. A yellow liquid was obtained (633 mg, quantitative yield).
¹H-NMR (CDCl₃): δ 8.05 - 8.02 (m, 1 H, Hₐᵣₒₘ) 7.97 - 7.78 (m, 3H, Hₐᵣₒₘ); 4.11 (q, 2H, O-CH₂CH₃, J = 7.1 Hz); 3.76 (t, 2H, N-CH₂, J = 7.5 Hz); 2.30 (t, 2H, CH₂-CO, J = 7.5 Hz); 1.86 (quin, 2H, CH₂); 1.69 (quin, 2H, CH₂); 1.49 - 1.39 (m, 2H, CH₂); 1.23 (t, 3H, CH₃, J= 7.4 Hz).
¹³C-NMR (CDCl₃): 173.5 (COOEt); 159.0 (CON); 137.9 (Cₐᵣₒₘ); 134.8 (CHₐᵣₒₘ); 134.4 (CHₐᵣₒₘ); 127.5 (Cₐᵣₒₘ); 125.2 (CHₐᵣₒₘ); 121.0 (CHₐᵣₒₘ); 60.3 (O-CH₂CH₃); 39.3 (N-CH₂); 34.2 (CH₂-CO); 28.2 (CH₂); 26.4 (CH₂); 24.5 (CH₂); 14.3 (CH₃).
HR LSIMS: Calculated for C₁₅H₁₉NO₅SNa (M+Na)⁺ 348.0882; found 348.0875.

### 1.2 Synthesis of 6-(1,1-dioxide-3-oxo-2H-benzo[d]isothiazol-2-yl)hexanoic acid (Step B)

The starting materials were the ethyl 6-(1,1-dioxide-3-oxo-2H-benzo[d]isothiazol-2-yl)hexanoate ester obtained in the previous step (625 mg, 1.92 mmol) and 6 ml of concentrated HCl. A white solid (387.6 mg) with m.p. = 98-100°C was obtained (Yield: 68%).
¹H-NMR (C₃D₆O): δ 10.45 (bs, 1 H, COOH); 8.15 - 7.97 (m, 4H, Hₐᵣₒₘ); 3.76 (t, 2H, N-CH₂, J = 7.3 Hz); 2.31 (t, 2H, CH₂-CO, J = 7.4 Hz); 1.84 (quin, 2H, CH₂); 1.66 (quin, 2H, CH₂); 1.52 - 1.42 (m, 2H, CH₂).
¹³C-NMR (C₃D₆O): 173.6 (COOH); 158.8 (CON); 138.0 (Cₐᵣₒₘ); 135.4 (CHₐᵣₒₘ); 134.9 (CHₐᵣₒₘ); 127.3 (Cₐᵣₒₘ); 125.0 (CHₐᵣₒₘ); 121.1 (CHₐᵣₒₘ); 38.8 (N-CH₂); 33.2 (CH₂-CO); 28.1 (CH₂); 26.1 (CH₂); 24.3 (CH₂).
HR LSIMS: Calculated for C₁₃H₁₅NO₅SNa (M+Na)⁺ 320.0569; found 320.0571

### 1.3 Synthesis of 6-(1,1-dioxide-3-oxo-2H-benzo[d]isothiazol-2-yl)-N-hydroxyhexanamide (MTC-97) (Steps C and D)

A Wang resin functionalized with hydroxylamine (0.14 mmol) was conditioned in dichloromethane (DCM) for 6 hours. It was then washed with dimethylformamide (DMF, 2x4ml) and a solution of HOAt (73.50 mg, 0.54 mmol), DIPCDI (0.08 ml, 0.54 mmol), and the 6-(1,1-dioxide-3-oxo-2H-benzo[d]isothiazol-2-yl)hexanoic acid obtained in the previous step (160.42 mg, 0.54 mmol) was added. After 24 hours of reaction, the resin was washed with DMF (3x4ml), and conditioned with DCM (3x4ml). The hydroxamic acid derivative bound to the resin was released from the resin by means of adding 10 ml of a solution of trifluoroacetic acid (TFA) in DCM at 50% for 30 minutes. Finally, the resin was washed with DCM and the organic phases were concentrated in the rotary evaporator. The obtained crude reaction product was purified by flash chromatography using DCM:MeOH (10:0.1) as an eluent. A beige solid with m.p. 139-141 °C was obtained.
¹H-NMR (DMSO-d₆): δ 10.31 (s, 1H, NHOH); 8.65 (s, 1H, OH); 8.28 (d,1H, Hₐᵣₒₘ, *J* = 7.2 Hz); 8.10 - 7.95 (m, 3H, Hₐᵣₒₘ); 3.67 (t, 2H, N-CH₂, *J* = 7.3 Hz); 1.92 (t, 2H, CH₂-CO, *J* = 7.3 Hz); 1.70 (quin, 2H, CH₂); 1.50 (quin, 2H, CH₂); 1.34 - 1.27 (m, 2H, CH₂).
¹³C-NMR (DMSO-d₆): 168.8 (CONHOH); 158.4 (CO); 136.7 (Cₐᵣₒₘ); 135.6 (CHₐᵣₒₘ); 135.1 (CHₐᵣₒₘ); 126.2(Cₐᵣₒₘ); 124.9 (CHₐᵣₒₘ); 121.3 (CHₐᵣₒₘ); 38.4 (N-CH₂); 31.9 (CH₂-CO); 27.5 (CH₂); 25.6 (CH₂); 24.4 (CH₂).
HR LSIMS: Calculated for C₁₃H₁ₗ₆N₂O0₅SNa (M+Na)⁺ 335.0677; found 335.0676

### Example 2. Synthesis of the inhibitor 7-(1,1-dioxide-3-oxo-2H-benzo[d]isothiazol-2-yl)-N-hydroxyheptanamide (MTC-124)

The synthesis comprised the following steps:

### 2.1 Synthesis of ethyl 7-(1 ,1-dioxide-3-oxo-2H-benzo[d]isothiazol-2-yl)heptanoate (Step A)

The starting materials were 1,1-dioxide-3-oxo-2H-benzo[d]isothiazole (400 mg, 1.95 mmol) and ethyl 7-bromoheptanoate (0.38 ml, 1.95 mmol) in 1 ml of DMF. It was purified by means of extracting with ethyl acetate (3x5ml), after adding ice-water. The organic phase was dried on MgSO₄, filtered and concentrated in the rotary evaporator. A yellow liquid was obtained (600 mg, yield = 91 %).
¹H-NMR (CDCl₃): δ 8.04 (dd, 1 H, Hₐᵣₒₘ, *J* = 2.2 Hz, J = 6.5 Hz) 7.92 - 7.78 (m, 3H, Hₐᵣₒₘ); 4.12 (q, 2H, O-CH₂CH₃, *J* = 7.1 Hz); 3.75 (t, 2H, N-CH₂, J = 7.5 Hz); 2.28 (t, 2H, CH₂-CO, J = 7.5 Hz); 1.84 (quin, 2H, CH₂); 1.63 (quin, 2H, CH₂); 1.44 - 1.36 (m, 4H, CH₂); 1.23 (t, 3H, CH₃, J = 7.2 Hz).
¹³C-NMR (CDCl₃): 173.7 (COOEt); 159.1 (CON); 137.9 (Cₐᵣₒₘ); 134.7 (CHₐᵣₒₘ); 134.3 (CHₐᵣₒₘ); 127.6 (Cₐᵣₒₘ); 125.2 (CHₐᵣₒₘ); 121.0 (CHₐᵣₒₘ); 60.3 (O-CH₂CH₃); 39.4 (N-CH₂); 34.3 (CH₂-CO); 28.6 (CH₂); 28.3 (CH₂); 26.5 (CH₂); 24.9 (CH₂); 14.3 (CH₃).
HR LSIMS: Calculated for C₁₆H₂₁NO₅SNa (M+Na)⁺ 362.1038; found 362.1031.

### 2.2 Synthesis of 7-(1,1-dioxide-3-oxo-2H-benzo[d]isothiazol-2-yl)heptanoic acid (Step B)

The starting materials were the ethyl 7-(1,1-dioxide-3-oxo-2H-benzo[*d*]isothiazol-2-yl)heptanoate ester obtained in the previous step (590 mg, 1.74 mmol) and 6 ml of concentrated HCl. A white solid (509 mg) with m.p. = 100-102°C was obtained (yield = 94%).
¹H-NMR (C₃D₆O): δ 8.15 - 7.95 (m, 4H, Hₐᵣₒₘ); 3.75 (t, 2H, N-CH₂, J = 7.4 Hz); 2.28 (t, 2H, CH₂-CO, J = 7.4 Hz); 1.81 (quin, 2H, CH₂, J = 7.3 Hz); 1.60 (quin, 2H, CH₂, *J* = 7.3 Hz); 1.47 - 1.38 (m, 4H, CH₂).
¹³C-NMR (C₃D₆O): 173.7 (COOH); 158.8 (CON); 138.0 (Cₐᵣₒₘ); 135.4 (CHₐᵣₒₘ); 134.9 (CHₐᵣₒₘ₎; 127.2 (Cₐᵣₒₘ); 125.0 (CHₐᵣₒₘ); 121.0 (CHₐᵣₒₘ); 38.9 (N-CH₂); 33.2 (CH₂-CO); 28.4 (CH₂); 28.2 (CH₂); 26.3 (CH₂); 24.6 (CH₂).
HR LSIMS: Calculated for C₁₄H₁₇NO₅SNa (M+Na)⁺ 334.0725; found 334.0728.

### 2.3 Synthesis of 7-(1,1-dioxide-3-oxo-2H-benzo[d]isothiazol-2-yl)-N-hydroxyheptanamide (MTC-124) (Steps C and D)

A Wang resin functionalized with hydroxylamine (0.14 mmol) was conditioned in dichloromethane (DCM) for 6 hours. It was then washed with dimethylformamide (DMF, 2x4ml) and a solution of HOAt (73.50 mg, 0.54 mmol), DIPCDI (0.08 ml, 0.54 mmol) and the 7-(1,1-dioxide-3-oxo-2H-benzo[*d*]isothiazol-2-yl)heptanoic acid obtained in the previous step (167.98 mg, 0.54 mmol) was added. After 24 hours of reaction, the resin was washed with DMF (3x4ml), and conditioned with DCM (3x4ml). The hydroxamic acid derivative bound to the resin was released from the resin by means of adding 10 ml of a solution of trifluoroacetic acid (TFA) in DCM at 50% for 30 minutes. Finally, the resin was washed with DCM and the organic phases were concentrated in the rotary evaporator. The crude reaction product obtained was purified by flash chromatography using DCM:MeOH (10:0.1) as an eluent. A light brown syrup was obtained (25 mg, yield = 42%).
¹H-NMR (CDCl₃): δ 8.04 (dd, 1 H, Hₐᵣₒₘ, *J* = 2.1 Hz, *J* = 6.6 Hz); 7.92 - 7.79 (m, 3H, Hₐᵣₒₘ); 3.75 (t, 2H, N-CH₂, *J* = 7.3 Hz); 2.14 (m, 2H, CH₂); 2.14 (m, 2H, CH₂); 1.82 (m, 2H, CH₂); 1.63 (m, 2H, CH₂); 1.40 (m, 2H, CH₂).
¹³C-NMR (CDCl₃): 159.2 (CONHOH); 137.7 (CO); 134.8 (Cₐᵣₒₘ); 134.8 (CHₐᵣₒₘ); 134.4 (CHₐᵣₒₘ); 127.4 (Cₐᵣₒₘ); 125.2 (CHₐᵣₒₘ); 121.0 (CHₐᵣₒₘ); 39.3 (N-CH₂); 29.8 (CH₂-CO); 28.3 (CH₂); 28.1 (CH₂); 26.2 (CH₂); 25.0 (CH₂).
HR LSIMS: Calculated for C₁₄H₁₈N₂O₅SNa (M+Na)⁺ 349.0834; found 349.0830.

### Example 3. Synthesis of the inhibitor 4-[(1,1-dioxide-3-oxo-2H-benzo[d]isothiazol-2-yl)methyl]-N-hydroxybenzamide (MTC-128)

The synthesis comprised the following steps:

### 3.1 Synthesis of ethyl 4-[(1,1-dioxide-3-oxo-2H-benzo[d]isothiazol-2-yl)methyl]benzoate (Step A)

The starting materials were 1,1-dioxide-3-oxo-2H-benzo[d]isothiazole (400 mg, 1.95 mmol) and ethyl 4-bromomethylbenzoate (474 mg, 1.95 mmol) in 1 ml of DMF. It was purified by means of extracting with ethyl acetate (3x5ml), after adding ice-water. The organic phase was dried on MgSO₄, filtered and concentrated in the rotary evaporator. The obtained product was used in the following reaction without more purification.

### 3.2 Synthesis of 4-[(1,1-dioxide-3-oxo-2H-benzo[d]isothiazol-2-yl)methyl]benzoic acid (Step B)

The starting materials were the ethyl 4-[(1,1-dioxide-3-oxo-2H-benzo[*d*]isothiazol-2-yl)methyl] benzoate ester obtained in the previous step (663 mg, 1.92 mmol) and 6 ml of concentrated HCl. The product was purified by flash chromatography using a mixture of DCM/hexane and was used in the following reaction without more purification.

### 3.3 Synthesis of 4-[(1,1-dioxide-3-oxo-2H-benzo[d]isothiazol-2-yl)methyl]-N-hydroxybenzamide (MTC-128) (Steps C and D)

A Wang resin functionalized with hydroxylamine (0.14 mmol) was conditioned in dichloromethane (DCM) for 6 hours. It was then washed with dimethylformamide (DMF, 2x4ml) and a solution of HOAt (74 mg, 0.54 mmol), DIPCDI (0.08 ml, 0.54 mmol) and the 4-[(1,1-dioxide-3-oxo-2H-benzo[*d*]isothiazol-2-yl)methyl] benzoic acid obtained in the previous step (171 mg, 0.54 mmol) was added. After 24 hours of reaction, the resin was washed with DMF (3x4ml) and conditioned with DCM (3x4ml). The hydroxamic acid derivative bound to the resin was released from the resin by means of adding 10 ml of a solution of trifluoroacetic acid (TFA) in DCM at 50% for 30 minutes. Finally, the resin was washed with DCM and the organic phases were concentrated in the rotary evaporator. The product obtained after the hydrolysis of the resin was recrystallized from a mixture of DCM/hexane. A beige solid was obtained.
¹H-NMR (DMSO-d₆): δ 11.20 (s, 1H, NHOH); 9.01 (s, 1H, OH); 8.33 (d, 1 H, Hₐᵣₒₘ , *J* = 6.0 Hz); 8.13 - 7.98 (m, 3H, Hₐᵣₒₘ); 7.71 (d, 1H, Hₐᵣₒₘ, *J* = 8.4 Hz); 7.47 (d, 1 H, Hₐᵣₒₘ, *J* = 8.4 Hz); 4.95 (s, 1H, CH₂).
HR LSIMS: Calculated for C₁₅H₁₂N₂O₅S (M)⁺ 332.0467; found 332.0468.

### Example 4. Synthesis of the inhibitor 6-[(benzo[d]isothiazol-3-yl)oxa]-N-hydroxyhexanamide (MTC-144)

The synthesis comprised the following steps:

### 4.1 Synthesis of ethyl 6-[(benzo[d]isothiazol-3-yl)oxa] hexanoate and ethyl 6-(3-oxo-2H-benzo[d]isothiazol-2-yl)hexanoate (Step A)

The starting material was a solution of benzo-[*d*]-isothiazol-3-(2H)-one (400 mg, 2.65 mmol) and K₂CO₃ (366 mg, 2.65 mmol) in acetonitrile (7 ml), to which ethyl 6-bromohexanoate (0.47 ml, 2.65 mmol) was added. It was taken to boiling for 24 hours. It was concentrated in the rotary evaporator, diluted in water, and an extraction with ethyl acetate (3x5ml) was performed. The organic phase was dried on MgSO₄, filtered and concentrated in the rotary evaporator. The two products formed were purified by means of flash chromatography using hexane:ethyl acetate (3:1) as an eluent. Two yellow liquids were obtained: ethyl 6-[(benzo[*d*]isothiazol-3-yl)oxa] hexanoate (yield: 426 mg, 55%) and ethyl 6-(3-oxo-2H-benzo[*d*]isothiazol-2-yl)hexanoate (yield: 245 mg, 32%).
Spectroscopic data of ethyl 6-[(benzo[*d*]isothiazol-3-yl)oxa] hexanoate:
¹H-NMR (CDCl₃): δ 7.89 (d, 1 H, Hₐᵣₒₘ, *J* = 8.0 Hz); 7.75 (d, 1H, Hₐᵣₒₘ, *J* = 8.2 Hz); 7.49 (ddd, 1 H, Hₐᵣₒₘ, *J* = 1.1 Hz, *J* = 7.0 Hz); 7.36 (ddd, 1H, Hₐᵣₒₘ, *J* = 0.8 Hz, *J* = 7.1 Hz); 4.53 (t, 2H, O-CH₂, *J* = 6.6 Hz); 4.12 (q, 2H, O-CH₂CH₃, *J* = 7.1 Hz); 2.34 (t, 2H, CH₂-CO, J = 7.5 Hz); 1.90 (quin, 2H, CH₂); 1.78 - 1.51 (m, 4H, CH₂); 1.24 (t, 3H, CH₃, *J* = 7.2 Hz).
¹³C-NMR (CDCl₃): 173.7 (COOEt); 163.3 (CO); 151.6 (Cₐᵣₒₘ); 128.7 (CHₐᵣₒₘ); 125.6 (Cₐᵣₒₘ); 124.4 (CHₐᵣₒₘ); 123.2 (CHₐᵣₒₘ); 120.2 (CHₐᵣₒₘ); 68.6 (O-CH₂); 60.3 (O-CH₂CH₃); 34.4 (CH₂-CO); 28.8 (CH₂); 25.7 (CH₂); 24.8 (CH₂); 14.3 (CH₃).
HR LSIMS: Calculated for C₁₅H₁₉NO₃SNa (M+Na)⁺ 316.0983; found 316.0979.
Spectroscopic data of ethyl 6-(3-oxo-2H-benzo[*d*]isothiazol-2-yl)hexanoate:
¹H-NMR (CDCl₃): δ 8.01 (d, 1 H, Hₐᵣₒₘ, *J*= 7.9 Hz); 7.61 - 7.51 (m, 2H, Hₐᵣₒₘ); 7.38 (m, 1H, Hₐᵣₒₘ); 4.09 (q, 2H, O-CH₂CH₃, *J* = 7.2 Hz); 3.88 (t, 2H, N-CH₂, *J* = 7.2 Hz); 2.28 (t, 2H, CH₂-CO, *J* = 7.4 Hz); 1.82 - 1.62 (m, 4H, CH₂); 1.45 - 1.37 (m, 2H, CH₂); 1.21 (t, 3H, CH₃, *J*= 7.2 Hz).
¹³C-NMR (CDCl₃)_{:} 173.5 (COOEt); 165.4 (CO); 140.2 (Cₐᵣₒₘ); 131.7 (CHₐᵣₒₘ); 126.7 (CHₐᵣₒₘ); 125.5 (CHₐᵣₒₘ); 124.8 (Cₐᵣₒₘ); 120.4 (CHₐᵣₒₘ); 60.3 (O-CH₂CH₃); 43.8 (N-CH₂); 34.2 (CH₂-CO); 29.3 (CH₂); 26.2 (CH₂); 24.6 (CH₂); 14.3 (CH₃).
HR LSIMS: Calculated for C₁₅H₁₉NO₃SNa (M+Na)⁺ 316.0983; found 316.0986.

### 4.2 Synthesis of 6-[(benzo[d]isothiazol-3-yl)oxa] hexanoic acid (Step B)

The starting materials were ethyl 6-[(benzo[*d*]isothiazol-3-yl)oxa] hexanoate obtained in the previous step (405 mg, 1.38 mmol) and 3.5 ml of concentrated HCl under reflux. It was concentrated in the rotary evaporator, water was added, and an extraction with ethyl acetate (3x5ml) was performed. The organic phase was dried on MgSO₄, filtered and concentrated in the rotary evaporator. It was purified by means of flash chromatography using hexane:ethyl acetate (3:1) as an eluent. The product (187 mg) was isolated with a yield = 51 %.
¹H-NMR (CDCl₃): δ 7.90 (d, 1 H, Hₐᵣₒₘ, *J* = 8.0 Hz); 7.75 (d, 1H, Hₐᵣₒₘ, *J* = 8.2 Hz); 7.50 (t, 1 H, Hₐᵣₒₘ); 7.36 (t, 1 H, Hₐᵣₒₘ, *J* = 7.5 Hz); 4.53 (t, 2H, O-CH₂, *J* = 6.5 Hz); 2.41 (t, 2H, CH₂-CO, *J* = 7.4 Hz); 1.95 - 1.86 (m, 2H, CH₂); 1.82 - 1.70 (m, 2H, CH₂); 1.61-1.52 (m, 2H, CH₂).
¹³C-NMR (CDCl₃): 179.4 (COOH); 163.3 (CO); 151.6 (Cₐᵣₒₘ); 128.7 (CHₐᵣₒₘ); 125.5 (Cₐᵣₒₘ); 124.4 (CHₐᵣₒₘ); 123.2 (CHₐᵣₒₘ); 120.2 (CHₐᵣₒₘ); 68.5 (O-CH₂); 39.9 (CH₂-CO); 28.8 (CH₂); 25.7 (CH₂); 24.5 (CH₂).

### 4.3 Synthesis of 6-[(benzo[d]isothiazol-3-yl)oxa]-N-hydroxyhexanamide (MTC-144) (Steps C and D)

A Wang resin functionalized with hydroxylamine (0.09 mmol) was conditioned in dichloromethane (DCM) for 6 hours. It was then washed with dimethylformamide (DMF, 2x4ml) and a solution of HOAt (49 mg, 0.36 mmol), DIPCDI (0.06 ml, 0.36 mmol) and the 6-[(benzo[*d*]isothiazol-3-yl)oxa] hexanoic acid obtained in the previous step (95 mg, 0.36 mmol) was added. After 24 hours of reaction, the resin was washed with DMF (3x4ml) and was conditioned with DCM (3x4ml). The hydroxamic acid derivative bound to the resin was released from the resin by means of adding 10 ml of a solution of trifluoroacetic acid (TFA) in DCM at 50% for 30 minutes. Finally, the resin was washed with DCM and the organic phases were concentrated in the rotary evaporator. The crude product was purified by means of flash chromatography using DCM:MeOH (10:0.3) as an eluent, a white solid being obtained (12 mg, yield = 24%).
¹H-NMR (DMSO-d₆): δ 10.32 (s, 1 H, NHOH); 8.65 (s, 1 H, OH); 8.04 (d, 1 H, Hₐᵣₒₘ, *J* = 8.2 Hz); 7.87 (d, 1H, Hₐᵣₒₘ, *J* = 8.0 Hz); 7.59 (dt, 1 H, Hₐᵣₒₘ, *J* = 1.1 Hz, *J* = 7.0 Hz, *J* = 8.1 Hz); 7.44 (dt, 1 H, Hₐᵣₒₘ, *J* = 0.8 Hz, *J* = 7.6 Hz, J = 8.1 Hz); 4.45 (t, 2H, O-CH₂, *J* = 6.6 Hz); 1.95 (t, 2H, CH₂-CO, *J* = 7.2 Hz); 1.79 (quin, 2H, CH₂); 1.55 (quin, 2H, CH₂); 1.44-1.34 (m, 2H, CH₂).
¹³C-NMR (DMSO-d₆): 168.9 (CONHOH); 162.4 (CO); 150.9 (Cₐᵣₒₘ); 128.9 (CHₐᵣₒₘ); 124.8 (CHₐᵣₒₘ); 124.5 (Cₐᵣₒₘ); 122.4 (CHₐᵣₒₘ); 120.9 (CHₐᵣₒₘ); 68.3 (O-CH₂); 32.0 (CH₂-CO); 28.0 (CH₂); 25.0 (CH₂); 24.7 (CH₂).
HR LSIMS: Calculated for C₁₃H₁₇N₂O₃S (M+H)⁺ 281.0960; found 281.0967.

### Example 5. Synthesis of the inhibitor 6-(3-oxo-2H-benzo[d]isothiazol-2-yl)-N-hydroxyhexanamide (MTC-136)

The synthesis comprised the following steps:

### 5.1. Synthesis of ethyl 6-(3-oxo-2H-benzo[d]isothiazol-2-yl)hexanoate (Step A)

It was performed as indicated in the previous section 4.1.

### 5.2. Synthesis of 6-(3-oxo-2H-benzo[d]isothiazol-2-yl)hexanoic acid (Step B)

The starting material was the ethyl 6-(3-oxo-2H-benzo[*d*]isothiazol-2-yl)hexanoate ester obtained in the previous step (234 mg, 0.80 mmol), it was treated with 3.5 ml of concentrated HCl under reflux. After the reaction time, it was diluted with water and the final precipitated product was filtered, and a white solid with m.p. = 59-61ºC was obtained. Yield: 177 mg (83%).
¹H-NMR (C₃D₆O): δ 10.47 (d, 1 H, COOH); 7.87 (q,2H, Hₐᵣₒₘ); 7.67 (ddd, 1 H, Hₐᵣₒₘ); 7.44 (ddd, 1H, Hₐᵣₒₘ); 3.87 (t, 2H, N-CH₂, *J* = 7.1 Hz); 2.29 (t, 2H, CH₂-CO, *J* = 7.4 Hz); 1.81 - 1.59 (m, 4H, CH₂); 1.43-1.35 (m, 2H, CH₂).
¹³C-NMR (C₃D₆O): 174.5 (COOH); 165.5 (CO); 141.4 (Cₐᵣₒₘ); 132.6 (CHₐᵣₒₘ); 126.8 (CHₐᵣₒₘ); 126.3 (CHₐᵣₒₘ); 125.6 (Cₐᵣₒₘ); 122.0 (CHₐᵣₒₘ); 43.9 (N-CH₂); 34.0 (CH₂-CO); 29.9 (CH₂); 26.7 (CH₂); 25.2 (CH₂).

### 5.3. Synthesis of 6-(3-oxo-2H-benzo[d]isothiazol-2-yl)-N-hydroxyhexanamide (MTC-136) (Steps C and D)

A Wang resin functionalized with hydroxylamine (0.09 mmol) was conditioned in dichloromethane (DCM) for 6 hours. It was then washed with dimethylformamide (DMF, 2x4ml) and a solution of HOAt (49 mg, 0.36 mmol), DIPCDI (0.06 ml, 0.36 mmol) and the 6-(3-oxo-2H-benzo[*d*]isothiazol-2-yl)hexanoic acid obtained in the previous step (105 mg, 0.36 mmol) was added. After 24 hours of reaction, the resin was washed with DMF (3x4ml), and was conditioned with DCM (3x4ml). The hydroxamic acid derivative bound to the resin was released from the resin by means of adding 10 ml of a solution of trifluoroacetic acid (TFA) in DCM at 50% for 30 minutes. Finally, the resin was washed with DCM and the organic phases were concentrated in the rotary evaporator. It was recrystallized from DCM. A white solid with m.p. = 153-155°C was obtained. Yield: 22 mg (44%).
¹H-NMR (DMSO-d₆): δ 10.30 (s, 1 H, NHOH); 7.94 (d, 1 H, Hₐᵣₒₘ, *J* = 8.1 Hz); 7.83 (d,1 H, Hₐᵣₒₘ, *J* = 7.8 Hz); 7.65 (t, 1 H, Hₐᵣₒₘ, *J* = 7.2 Hz); 7.41 (t, 1 H, Hₐᵣₒₘ, *J* = 7.4 Hz); 3.78 (t, 2H, N-CH₂, *J* = 7.0 Hz); 1.90 (t, 2H, CH₂-CO, *J =* 7.3 Hz); 1.68 - 1.44 (m, 4H, CH₂); 1.28 - 1.20 (m, 2H, CH₂).
¹³C-NMR (DMSO-d₆): 168.8 (CONHOH); 164.2 (CO); 140.2 (Cₐᵣₒₘ); 131.6 (CHₐᵣₒₘ); 125.5 (CHₐᵣₒₘ); 125.4 (CHₐᵣₒₘ); 124.1 (Cₐᵣₒₘ); 121.8 (CHₐᵣₒₘ); 42.7 (N-CH₂); 32.0 (CH₂-CO); 28.6 (CH₂); 25.5 (CH₂); 24.6 (CH₂).

### Example 6

The inhibitory activity of the compounds obtained in the previous Examples 1, 2, 3 and 5 was evaluated, for which the HDAC AK-501 colorimetric titration kit for inhibition, supplied by Biomol, was used, following the indicated protocol.

The process of the assay was carried out in two steps. In the first step, the Color de Lys^{®} substrate, containing an acetylated lysine group, was incubated with a sample of HeLa (human cervical cancer cell line) nuclear extract, rich in HDAC activity. In the second step, the previous mixture was treated with the Color de Lys^{®} developer, which caused an increase in the quantifiable color intensity at 405 nm. There is a linear correlation between the absorption and the deacetylation of lysine within instrumental limits. The addition of an HDAC inhibitor gives rise to a lower deacetylation of the Color de Lys^{®} substrate and to a decrease in the intensity at 405 nm.

The operating procedure was the following:
1. The buffer, diluted (250 nM) TSA (Trichostatin A) and the inhibitor to be assayed were added at the desired concentrations in the suitable wells of the plate (See Table below).
2. The Hela extract was added, except to the enzyme-free control.
3. The plate and the Color de Lys^{®} substrate were thermostatted at 37ºC.
4. The reactions were started adding the 0.4 mM substrate (final concentration of the substrate 0.2 mM) in each well and it was stirred.
5. A reaction time of 20 minutes at 37ºC was allowed.
6. The Color de Lys^{®} developer, prepared approximately 30 minutes before its use, was added (the developer was used diluted 20 times with an amount of TSA resulting in a final concentration of 1µM in the assay). It was allowed to react for 15 minutes at 37 ºC.
7. The reading was made at 405 nm.

**Table**

| **Wells** | **Butter** | **HeLa** | **Inhibitor (x5)** | **Substrate (x2)** | **Developer** |
|---|---|---|---|---|---|
| **Blank** | 25 µl | 0 | 0 | 25 µl | 50 µl |
| **Control** | 20 µl | 5 µl | 0 | 25 µl | 50 µl |
| **TSA** | 10 µl | 5 µl | 10 µl | 25 µl | 50 µl |
| **Inhibitor** | 10 µl | 5 µl | 10 µl | 25 µl | 50 µl |

| | | | | | |
|---|---|---|---|---|---|
| (x5) and (x2) indicate the dilution factors. | | | | | |

The result of the reading using 5 different concentrations taken between 0.1-2.0 µM of each inhibitor allowed obtaining a straight line of concentrations versus enzymatic activity. The concentration necessary to deduce the 50% enzymatic activity (IC₅₀) was calculated from the equation of said line. The compounds with values of IC₅₀ less than 10 µM were considered significant from the biological point of view.

| **Compound** | **Reference** | **IC₅₀** |
|---|---|---|
| 1 | MTC-97 | 1.5 µM |
| 2 | MTC-124 | 0.25 µM |
| 3 | MTC-128 | 1.15 µM |
| 5 | MTC-144 | 0.21 µM |

The results obtained of IC₅₀ for the four compounds shown in the previous table show that the derivatives of benzo[d]isothiazole are useful as HDAC inhibitors.

No derivative of this nature has been previously described as an HDAC inhibitor. Furthermore, the activity is modulated: a) by the nature of the spacer between the benzo[d]isothiazole fragment and the hydroxamic acid function, and b) by the degree of oxidation of the sulfur in the benzo[d]isothiazole ring and the position of the spacer on the benzo[d]isothiazole ring (N-derivatives and O-derivatives).

## Claims

1. A histone deacetylase inhibitor compound selected from among compounds of general formula (Ia) and (Ib) 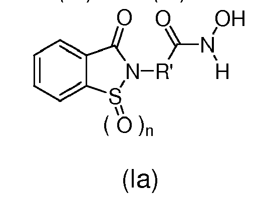 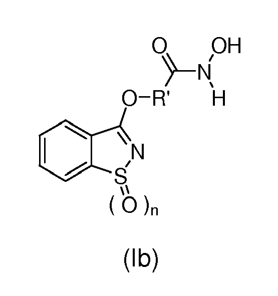 wherein
R' represents a -(CH₂)ₘ- radical, wherein m is 4, 5 or 6, or a 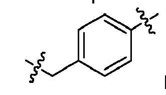 radical, and
n is 0 or 2,
optionally in the form of one of the salts thereof, particularly one of the pharmaceutically acceptable salts thereof, or one of the corresponding solvates thereof.

2. A compound according to claim 1 of formula (Ia), wherein R' represents a -(CH₂)ₘ- radical, wherein m is 5 or 6, or a 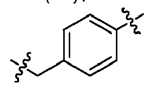 radical,
and n is 0 or 2.

3. A compound according to claim 2, wherein R' represents a - (CH₂)ₘ- radical, wherein m is 5 or 6 and n is 0 or 2.

4. A compound according to claim 3, wherein R' represents a - (CH₂)ₘ- radical, wherein m is 5 or 6 and n is 2.

5. A compound according to claim 2, wherein R' represents a 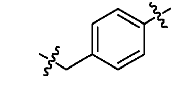 radical, and n is 0 or 2.

6. A compound according to claim 1 of formula (Ib), wherein R' represents a -(CH₂)ₘ- radical, wherein m is 4 or 5, or a 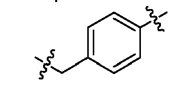 radical, and n is 0 or 2.

7. A compound according to claim 6, wherein R' represents a - (CH₂)ₘ- radical, wherein m is 4 or 5 and n is 0 or 2.

8. A compound according to claim 7, wherein R' represents a - (CH₂)ₘ- radical, wherein m is 4 or 5 and n is 0.

9. A compound according to claim 6, wherein R' represents a 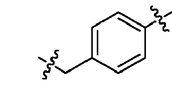 radical, and n is 0 or 2.

10. A compound according to claim 1 selected from the following group:
[1] 6-(1,1-dioxide-3-oxo-2H-benzo[*d*]isothiazol-2-yl)-N-hydroxyhexanamide (MTC-97)
[2] 7-(1,1-dioxide-3-oxo-2H-benzo[*d*]isothiazol-2-yl)-N-hydroxyheptanamide (MTC-124)
[3] 4-[(1,1-dioxide-3-oxo-2H-benzo[*d*]isothiazol-2-yl)methyl]-*N-*hydroxybenzamide (MTC-128)
[4] 6-(3-oxo-2H-benzo[*d*]isothiazol-2-yl)-*N-*hydroxyhexanamide (MTC-136)
[5] 6-[(benzo[*d*]isothiazol-3-yl)oxa]-*N-*hydroxyhexanamide (MTC-144)

11. A pharmaceutical composition containing one or more histone deacetylase inhibitor compounds of general formula (Ia) or (Ib), or one of the pharmaceutically acceptable salts thereof, or one of the corresponding solvates thereof according to any of the previous claims and one or more pharmaceutically acceptable excipients.

12. A histone deacetylase inhibitor compound of general formula (Ia) or (Ib) or one of the pharmaceutically acceptable salts thereof, or one of the corresponding solvates thereof according to any of claims 1 to 10, for the treatment and/or prophylaxis of diseases sensitive to the inhibition of the histone deacetylases in mammals, including man.

13. A histone deacetylase inhibitor compound of general formula (Ia) or (Ib) or one of the pharmaceutically acceptable salts thereof, or one of the corresponding solvates thereof according to any of claims 1 to 10, for the treatment and/or prophylaxis of cancer, particularly leukemia, solid and altered hormonal signal-dependent tumors, psoriasis, inflammatory type diseases, the infection caused by HIV, Alzheimer's disease and senile dementia in mammals, including man.

14. The use of a histone deacetylase inhibitor compound of general formula (Ia) or (Ib) or one of the pharmaceutically acceptable salts thereof, or one of the corresponding solvates thereof according to any of claims 1 to 10, in the production of a medicament for the treatment and/or prophylaxis of diseases sensitive to the inhibition of histone deacetylases in mammals, including man.

15. The use of a histone deacetylase inhibitor compound of general formula (Ia) or (Ib) or one of the pharmaceutically acceptable salts thereof, or one of the corresponding solvates thereof according to any of claims 1 to 10, in the production of a medicament for the treatment and/or prophylaxis of cancer, particularly leukemia, solid and altered hormonal signal-dependent tumors, psoriasis, inflammatory type diseases, infection caused by HIV, Alzheimer's disease and senile dementia in mammals, including man.

16. A process for preparing a histone deacetylase inhibitor compound of general formula (Ia) 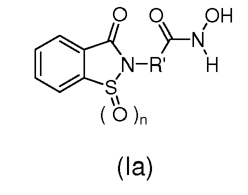 wherein
R' represents a -(CH₂)ₘ- radical wherein m is 5 or 6; and n is 0 or 2
comprising the following reaction steps:
step A: 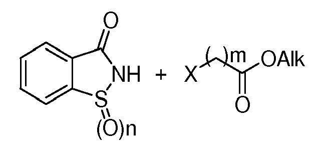 comprising reacting a derivative of benzo[d]isothiazole wherein n is 0 or 2, with an ester derivative compound wherein
m is 5 or 6;
X represents a leaving group, selected from Br, Cl, -OSO₂CH₃ and -OSO₂Ph(pCH₃); and
Alk represents an alkyl group selected from ethyl, methyl and t-butyl, or an acid function protecting group;
in the presence of an inorganic base and in an inert solvent;
step B comprising the hydrolysis of the ester derivative obtained in step A of general formula 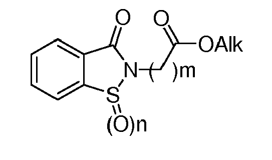 wherein n, Alk and m have the aforementioned meaning,
or alternatively, the removal of the acid function protecting group to obtain the corresponding carboxylic acid derivative of general formula: 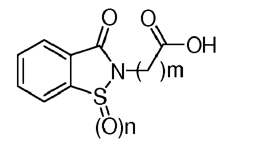 wherein n and m have the aforementioned meaning,
step C comprising contacting a Wang resin functionalized with hydroxylamine, with hydroxyazabenzotriazole (HOAt), diisopropylcarbodiimide (DPICDI) and the carboxylic acid derivative obtained in step B to obtain the corresponding hydroxamic acid derivative bound to the Wang resin of general formula: 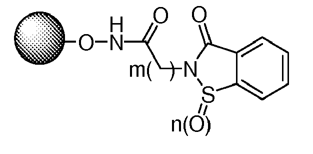 wherein n and m have the aforementioned meaning; and
step D comprising the release of the hydroxamic acid derivative bound to the Wang resin obtained in step C to obtain a compound of general formula (Ia) 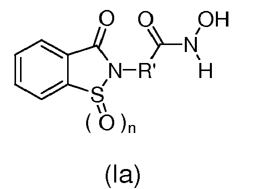 wherein R' and n have the previously defined meaning.

17. A process for preparing a histone deacetylase inhibitor compound of general formula (Ia) 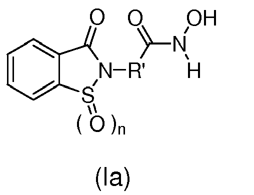 wherein R' is a 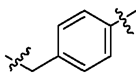 radical and
n is 0 or 2
comprising the following reaction steps:
step A : 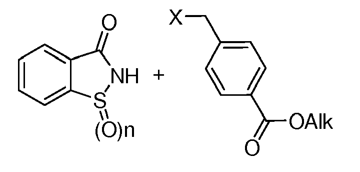 comprising reacting a derivative of benzo[d]isothiazole, wherein n is 0 or 2, with an ester derivative compound wherein
X represents a leaving group selected from Br, Cl, -OSO₂CH₃ and -OSO₂Ph(pCH₃); and
Alk represents an alkyl group selected from ethyl, methyl and t-butyl, or an acid function protecting group;
in the presence of an inorganic base and in an inert solvent;
step B comprising the hydrolysis of the ester derivative obtained in step A of general formula: 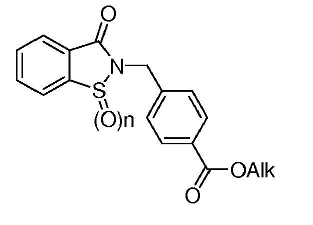 wherein n and Alk have the aforementioned meaning,
or alternatively, the removal of the acid function protecting group to obtain the corresponding carboxylic acid derivative of general formula: 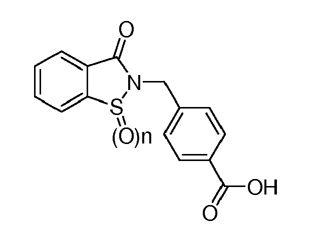 wherein n has the aforementioned meaning,
step C comprising contacting a Wang resin functionalized with hydroxylamine, with hydroxyazabenzotriazole (HOAt), diisopropylcarbodiimide (DPICDI) and the carboxylic acid derivative obtained in step B to obtain the corresponding hydroxamic acid derivative bound to the Wang resin of general formula: 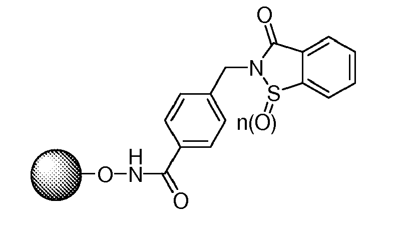 wherein R has the aforementioned meaning; and
step D comprising the release of the hydroxamic acid derivative bound to the Wang resin obtained in step C to obtain a compound of general formula (Ia) 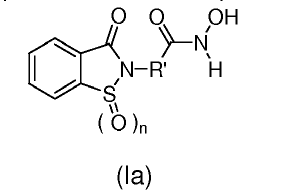 wherein R' and n have the previously defined meaning.

18. A process for preparing a histone deacetylase inhibitor compound of general formula (Ib) 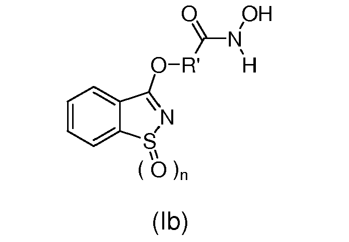 wherein
R' represents a -(CH₂)ₘ- radical wherein m is 4 or 5; and n is 0,
comprising the following reaction steps:
step A: 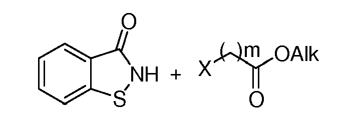 comprising reacting a derivative of benzo[d]isothiazole,
with an ester derivative compound wherein
m is 4 or 5;
X represents a leaving group, selected from Br, Cl, -OSO₂CH₃ and -OSO₂Ph(pCH₃); and
Alk represents an alkyl group selected from ethyl, methyl and t-butyl, or an acid function protecting group;
in the presence of an inorganic base and in an inert solvent;
step B comprising the hydrolysis of the ester derivative obtained in step A of general formula 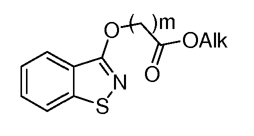 wherein Alk and m have the aforementioned meaning
or alternatively, the removal of the acid function protecting group to obtain the corresponding carboxylic acid derivative of general formula: 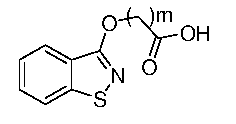 wherein m has the aforementioned meaning,
step C comprising contacting a Wang resin functionalized with hydroxylamine, with hydroxyazabenzotriazole (HOAt), diisopropylcarbodiimide (DPICDI) and the carboxylic acid derivative obtained in step B to obtain the corresponding hydroxamic acid derivative bound to the Wang resin of general formula: 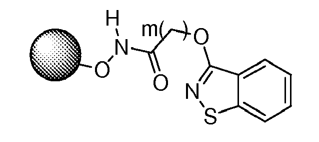 wherein m has the aforementioned meaning; and
step D comprising the release of the hydroxamic acid derivative bound to the Wang resin obtained in step C to obtain a compound of general formula (Ib) 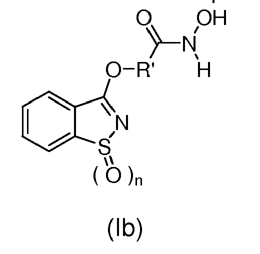 wherein R' and n have the previously defined meaning.

19. A process for preparing a histone deacetylase inhibitor compound of general formula (Ib) 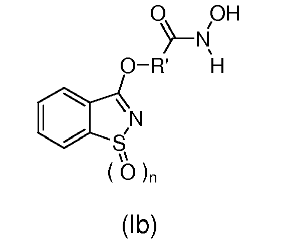 wherein
R' represents a -(CH₂)ₘ- radical wherein m is 4 or 5; and n is 2,
comprising the following reaction steps:
step A: 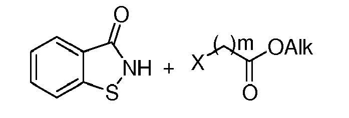 comprising reacting a derivative of benzo[d]isothiazole,
with an ester derivative compound wherein
m is 4 or 5;
X represents a leaving group, selected from Br, Cl, -OSO₂CH₃ and -OSO₂Ph(pCH₃); and
Alk represents an alkyl group selected from ethyl, methyl and t-butyl, or an acid function protecting group;
in the presence of an inorganic base and in an inert solvent
step A' comprising the oxidation of the ester obtained in step A, or alternatively the product with the acid function protected, of general formula 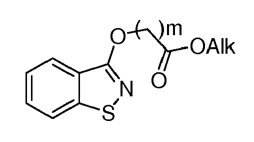 wherein m and Alk have the aforementioned meaning,
in the presence of a solution of H₅lO₆ and CrO₃ in acetonitrile and at -35ºC,
step B comprising the hydrolysis of the oxidized ester derivative obtained in step A' of general formula 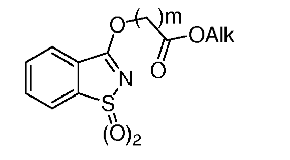 wherein Alk and m have the aforementioned meaning
or alternatively, the removal of the acid function protecting group to obtain the corresponding carboxylic acid derivative of general formula: 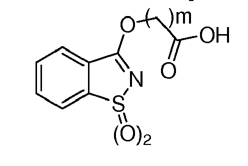 wherein m has the aforementioned meaning,
step C comprising contacting a Wang resin functionalized with hydroxylamine, with hydroxyazabenzotriazole (HOAt), diisopropylcarbodiimide (DPICDI) and the carboxylic acid derivative obtained in step B to obtain the corresponding hydroxamic acid derivative bound to the Wang resin of general formula: 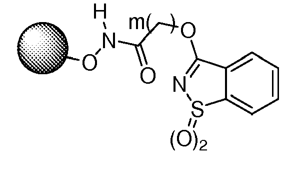 wherein m has the aforementioned meaning; and
step D comprising the release of the hydroxamic acid derivative bound to the Wang resin obtained in step C to obtain a compound of general formula (Ib) 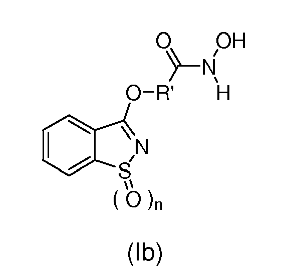 wherein R' and n have the previously defined meaning.

20. A process for preparing a histone deacetylase inhibitor compound of general formula (Ib) 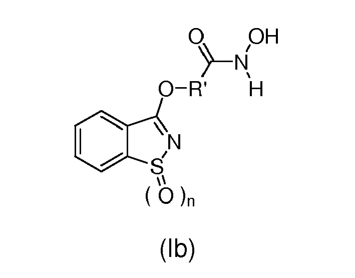 wherein
R' represents a 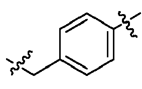 radical and n is 0,
comprising the following reaction steps:
step A: 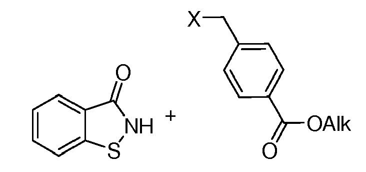 comprising reacting a derivative of benzo[d]isothiazole,
with an ester derivative compound wherein
X represents a leaving group selected from Br, Cl, -OSO₂CH₃ and -OSO₂Ph(pCH₃); and
Alk represents an alkyl group selected from ethyl, methyl and t-butyl, or an acid function protecting group;
in the presence of an inorganic base and in an inert solvent;
step B comprising the hydrolysis of the ester derivative obtained in step A of general formula 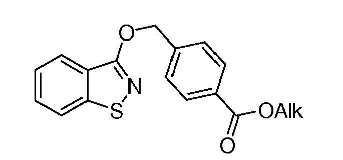 wherein Alk has the aforementioned meaning
or alternatively, the removal of the acid function protecting group to obtain the corresponding carboxylic acid derivative of general formula: 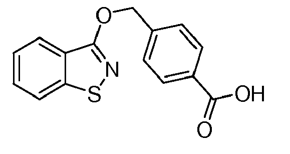 step C comprising contacting a Wang resin functionalized with hydroxylamine, with hydroxyazabenzotriazole (HOAt), diisopropylcarbodiimide (DPICDI) and the carboxylic acid derivative obtained in step B to obtain the corresponding hydroxamic acid derivative bound to the Wang resin of general formula: 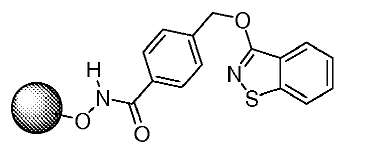 and
step D comprising the release of the hydroxamic acid derivative bound to the Wang resin obtained in step C to obtain a compound of general formula (Ib) 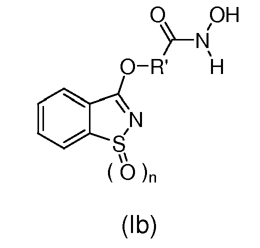 wherein R' and n have the previously defined meaning.

21. A process for preparing a histone deacetylase inhibitor compound of general formula (Ib) 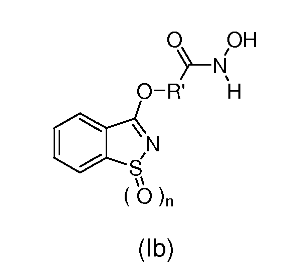 wherein
R' represents a 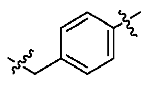 radical and n is 2,
comprising the following reaction steps:
step A: 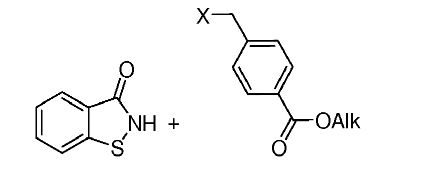 comprising reacting a derivative of benzo[d]isothiazole,
with an ester derivative compound wherein
X represents a leaving group, selected from Br, Cl, -OSO₂CH₃ and -OSO₂Ph(pCH₃); and
Alk represents an alkyl group selected from ethyl, methyl and t-butyl, or an acid function protecting group;
in the presence of an inorganic base and in an inert solvent
step A' comprising the oxidation of the ester obtained in step A, or alternatively the product with the acid function protected, of general formula 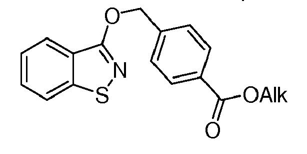 wherein Alk has the aforementioned meaning,
in the presence of a solution of H₅lO₆ and CrO₃ in acetonitrile and at -35ºC,
step B comprising the hydrolysis of the oxidized ester derivative obtained in step A' of general formula 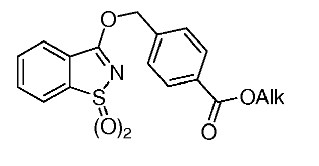 wherein Alk has the aforementioned meaning
or alternatively, the removal of the acid function protecting group to obtain the corresponding carboxylic acid derivative of general formula: 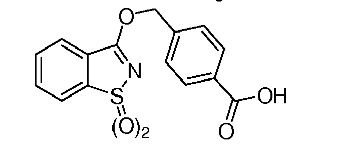 step C comprising contacting a Wang resin functionalized with hydroxylamine, with hydroxyazabenzotriazole (HOAt), diisopropylcarbodiimide (DPICDI) and the carboxylic acid derivative obtained in step B to obtain the corresponding hydroxamic acid derivative bound to the Wang resin of general formula: 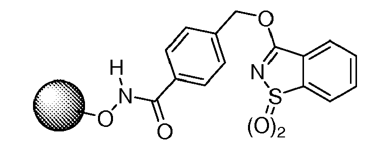 and
step D comprising the release of the hydroxamic acid derivative bound to the Wang resin obtained in step C to obtain a compound of general formula (Ib) 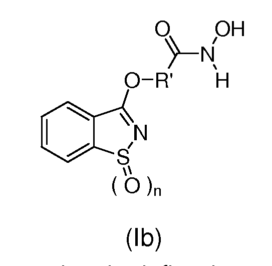 wherein R' and n have the previously defined meaning.
